(19) Europäisches Patentamt
European Patent Office
Office européen des brevets

(11) Numéro de publication : **0 208 641 B1**

# (12) FASCICULE DE BREVET EUROPÉEN

(45) Date de publication du fascicule du brevet : **20.09.89**

(51) Int. Cl.$^4$ : **B 01 J 19/00**, C 07 K 1/04

(21) Numéro de dépôt : **86440041.1**

(22) Date de dépôt : **28.05.86**

(54) **Multi-synthétiseur et procédé de production de peptides de synthèse semi-automatique en phase solide.**

(30) Priorité : **03.06.85 FR 8508438**

(43) Date de publication de la demande : **14.01.87 Bulletin 87/03**

(45) Mention de la délivrance du brevet : **20.09.89 Bulletin 89/38**

(84) Etats contractants désignés : **AT BE CH DE FR GB IT LI LU NL SE**

(56) Documents cités :
**EP–A– 0 042 792**
**EP–A– 0 130 739**
**WO–A–85 /012 24**
**FR–A– 2 554 820**
**US–A– 3 557 077**
**US–A– 3 715 190**
**JOURNAL OF CHROMATOGRAPHIC SCIENCE, vol. 10, no. 6, juin 1972, pages 384-392, Niles, Illinois, US; R.P.W. SCOTT et al.: "An automatic apparatus for the synthesis of peptides using resin coated glass beads in the form of a packed bed"**

(73) Titulaire : **CENTRE NATIONAL DE LA RECHERCHE SCIENTIFIQUE (CNRS)
15, Quai Anatole France
F-75007 Paris (FR)**

(72) Inventeur : **Neimark, Jean
10 avenue du Général de Gaulle
F-67000 Strasbourg (FR)**
Inventeur : **Briand, Jean-Paul
22 rue des Balayeurs
F-67000 Strasbourg (FR)**

(74) Mandataire : **Nuss, Pierre
10, rue Jacques Kablé
F-67000 Strasbourg (FR)**

**EP 0 208 641 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention, réalisée par le laboratoire LP 6201 de l'Institut de Biologie Moléculaire et Cellulaire du Centre National de la Recherche Scientifique (CNRS), concerne la synthèse chimique de peptides, et a pour objet un multi-synthétiseur de peptides comportant des moyens pour assurer la synthèse simultanée d'un nombre variable de peptides en quantité différente et de séquences identiques ou différentes.

Actuellement, aucun des appareils existants ne permet la synthèse simultanée de plus de deux peptides de séquences différentes. Les synthétiseurs qui existent sont soit des mono-synthétiseurs, soit des bi-synthétiseurs, manuels, semi-automatiques ou automatiques. Les mono-synthétiseurs ne sont capables de synthétiser qu'un seul peptide à la fois et les bi-synthétiseurs, qui mettent deux réacteurs en œuvre, ne peuvent synthétiser simultanément que deux peptides différents. Or, la synthèse d'un peptide de 20 à 30 amino-acides avec vérification de chaque couplage d'amino-acide nécessite généralement une dizaine de jours. Avec de tels appareils, on ne peut donc raisonnablement envisager la production que de quatre peptides synthétiques par mois. De plus, il faut noter qu'aucun des appareils automatiques actuellement sur le marché n'effectue des tests de contrôle aux stades essentiels de la synthèse.

La présente invention a pour but de permettre simultanément la synthèse d'un nombre variable de peptides de séquences identiques ou différentes suivant une technique de synthèse commune.

Elle a en effet pour objet un multi-synthétiseur de peptides semi-automatique en phase solide, caractérisé en ce qu'il est principalement constitué par des réacteurs chimiques reliés à des doseurs volumétriques de solvant disposés en amont desdits réacteurs chimiques et en aval d'un sélecteur de solvant alimenté en solvant grâce à des bouteilles de solvant sous pression d'azote, par des cylindres de distribution, ainsi que par une centrale de réglage et de régulation d'azote très basse pression, ces moyens étant séquentiellement commandés par un automate programmable ou un micro-ordinateur assurant la mise en œuvre de la technique de synthèse, et le nombre maximum d'ensembles doseur volumétrique de solvant-réaction chimique étant égal au nombre maximum de peptides pouvant être fabriqués simultanément.

Durant ces dernières années, la synthèse chimique de peptides a pris un essor considérable. Il s'est avéré en effet, que de nombreux peptides jouaient un rôle fondamental dans la communication entre neurones. L'utilisation de peptides de synthèse a alors permis des progrès rapides dans la compréhension du mécanisme d'action des neuropeptides et dans leur utilisation en thérapie.

Mais l'utilisation de peptides synthétiques a également pris une place prépondérante dans plusieurs autres domaines. En immunologie, cette technologie constitue une voie d'avenir dans l'obtention de vaccins synthétiques. On synthétise, dans ce cas, des peptides correspondant à des régions antigéniques de protéines virales. Des résultats récents montrent que les peptides couplés, injectés à l'animal, peuvent le protéger de l'infection virale. L'utilisation de tels vaccins synthétiques permettra d'éviter les problèmes inhérents à la fabrication de vaccins classiques, notamment la persistance accidentelle d'agents pathogènes dans les préparations inactivées de virus, et l'injection d'acide nucléique viral toujours présent dans ces préparations.

En biologie moléculaire, grâce aux développements du génie génétique, il est possible de disposer relativement facilement de la séquence de gènes codant pour des protéines fonctionnelles ou des protéines de structure. On décode alors ces séquences nucléotidiques et on obtient les séquences en acides aminés de ces protéines sans avoir eu, ni à les isoler, ni à les purifier. Il est cependant nécessaire de savoir si ces protéines sont réellement exprimées dans le système cellulaire étudié. C'est à ce niveau qu'intervient l'utilisation de peptides de synthèse. On synthétise les fragments peptidiques précités comme étant des déterminants antigéniques probables de la protéine étudiée, on les couple à une protéine porteuse avant d'être injectés à l'animal. Etant donné que des anticorps anti-peptides sont capables, dans la plupart des cas, de reconnaître la protéine native, on peut alors savoir si la protéine est exprimée ou non dans la cellule. On peut aussi, à l'aide de colonnes d'immunoadsorption, envisager de purifier la protéine en question à partir d'un extrait brut cellulaire. Il est également possible de suivre, par exemple, le destin de divers domaines d'une protéine lorsque celle-ci subit un processus de maturation.

Différentes techniques de synthèse sont connues. La technique la plus couramment utilisée est celle développée par Merrifield. Son principe est le suivant :

Les amino-acides sont ajoutés séquentiellement sur la chaîne en croissance dont l'extrémité C-terminale est liée à un support solide insoluble. Toutes les réactions ont lieu dans un même récipient et les excès de réactifs sont éliminés par simples filtrations et lavages. Le déroulement des opérations s'effectue de la manière suivante :

(Voir Schéma page 3)

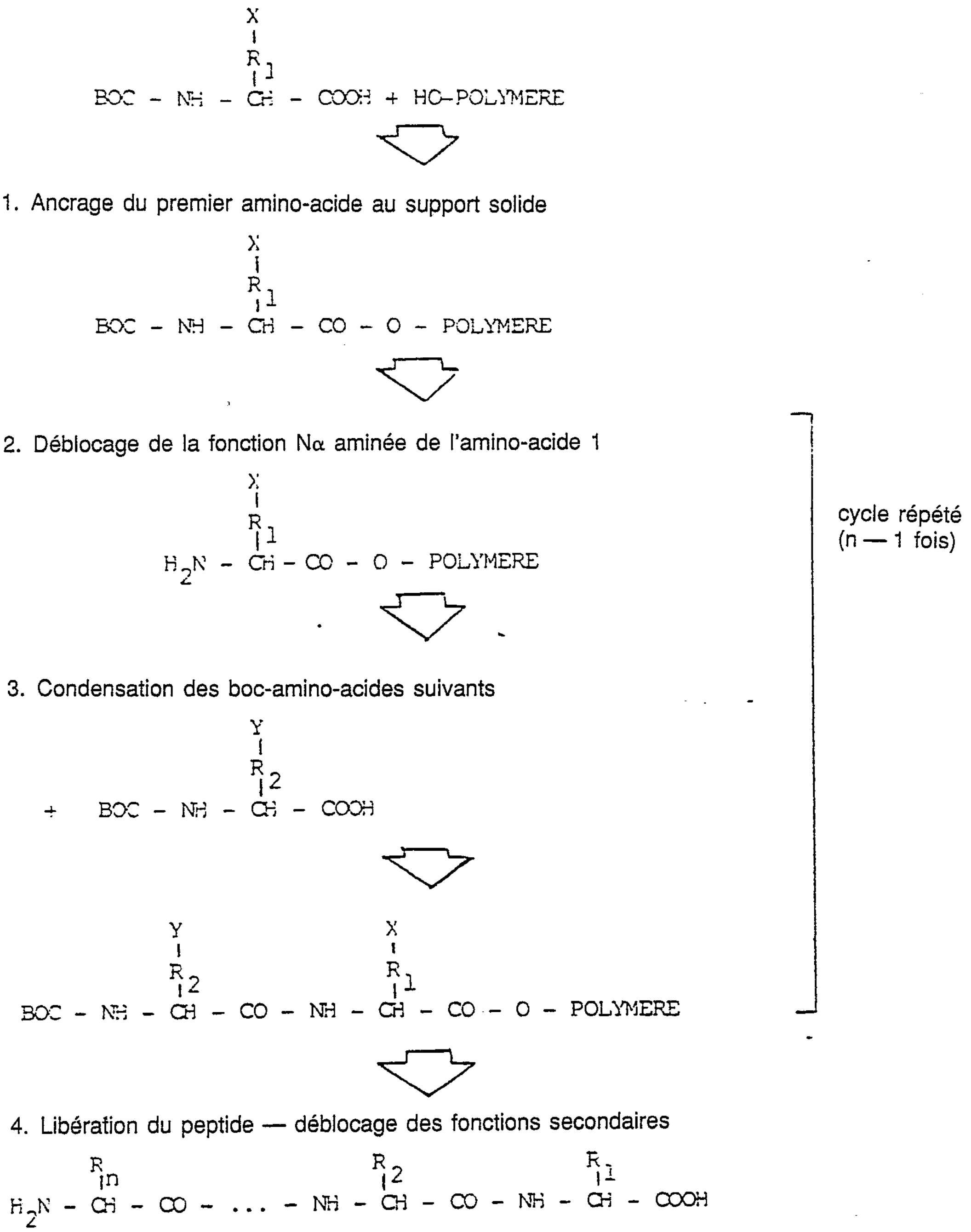


Le support solide est une résine de polystyrène polymérisée avec 1 % de divinylbenzène et portant un grand nombre de groupements susceptibles de réagir avec la fonction —COOH des acides aminés. Pendant l'étape de condensation, la fonction Nα aminée de l'amino-acide ajouté doit être bloquée : le groupement protecteur le plus utilisé à l'heure actuelle est le t-butyloxycarbonyle (BOC). Les groupements fonctionnels secondaires sont également protégés durant tout le temps de la synthèse. Ils ne seront débloqués que lors de la libération du peptide de la résine par l'acide fluorhydrique.

Le groupement t-butyloxycarbonyle est éliminé par acidolyse dans un système acide trifluoroacétique-dichlorométhane contenant un agent anti-oxydant. Le groupement aminé obtenu sous forme de sel est alors neutralisé en présence d'une base telle que la N,N diisopropyléthylamine. Ces deux principales étapes sont séparées par plusieurs étapes de lavage. Puis on vérifie la réaction de déprotection par un test à la ninhydrine.

L'agent de condensation utilisé est la N,N' dicyclohexylcardodiimide qui active le groupement carboxyle du groupement protecteur t-butyloxycarbonyle-amino-acide à ajouter, en O-acyl urée. Cet intermédiaire peut aussi s'isomériser rapidement en N-acyl urée inactive.

Les conditions de réaction sont les suivantes :

* groupement t-butyloxycarbonyle-amino-acide : excès molaire × 3

* N,N' dicyclohexylcarbodiimide du dichlorométhane pendant 1 à 2 heures.

Des variantes de la réaction de couplage consistent à ajouter l'amino-acide sous forme préactivée. Les deux formes les plus utilisées sont les esters activés ou les anhydrides symétriques d'amino-acides.

Quelle que soit la méthode de couplage utilisée, on vérifie la réaction de condensation par le test à la ninhydrine. Si la réaction de couplage n'est pas complète on effectue une double condensation, c'est-à-dire que l'on répète l'étape de neutralisation. Il se peut, en effet, qu'un faible pourcentage de peptide-résine soit resté sous forme de sel. On ajoute ensuite le groupement t-butyloxycarbonyle-amino-acide et la N,N' dicyclohexylcarbodiimide en excès 1,5 molaire pendant 30 mn à 1 heure.

Si le test ninhydrique reste toujours légèrement positif, on bloque, par une réaction d'acétylation, les groupements N aminés restés libres qui pourraient donner lieu à des réactions d'addition lors de condensations ultérieures.

* anhydrique acétique : excès molaire × 10
* N,N diisopropyléthylamine : excès molaire × 11

dans du dichlorométhane pendant 10 mn.

Il existe une variante de la technique de Merrifield mise au point par Sheppard. Le principe général est le même, seuls changent la nature des groupements protecteurs, les solvants de déprotection et éventuellement la nature de la résine.

Merrifield a élaboré cette technique de synthèse dans le but de permettre son automatisation. Certes, la qualité du produit final, c'est-à-dire celle du peptide obtenu avec un appareil de synthèse automatique, semi-automatique ou manuel est la même quelle que soit la conception dudit appareil, mais l'utilisation d'un appareil manuel de synthèse implique une servitude telle que la présence permanente du manipulateur est indispensable. Cependant, l'utilisation d'un appareil entièrement automatique ne permet pas le contrôle immédiat de la qualité de chaque étape fondamentale de la synthèse et c'est pourquoi la présente invention concerne un multi-synthétiseur de peptides semi-automatique visant à l'obtention d'un produit final de très haute pureté.

Le document WO-A-85/01224 a pour objet un synthétiseur de peptides. Mais, dans ce dernier, les différents solvants et réactifs sont tous acheminés à travers une seule et même ligne de distribution, ce qui ne permet pas, malgré toutes les différentes procédures de lavage prévues, de décontaminer efficacement l'unique ligne de distribution, altérant ainsi inévitablement la pureté du peptide obtenu. De plus, l'activation du milieu résine-amino-acide est effectuée par un système à rotation mécanique, ce qui entraîne, d'une part, des risques d'altération de la résine elle-même, et, d'autre part, une consommation accrue des solvants et des réactifs.

L'invention sera mieux comprise, grâce à la description ci-après, qui se rapporte à des modes de réalisation préférés, donnés à titre d'exemples non limitatifs, et expliqués avec référence aux dessins schématiques annexés, dans lesquels :

la figure 1 est une vue en perspective du multi-synthétiseur de peptides conforme à l'invention ;
la figure 2 est une vue en coupe d'un doseur volumétrique ;
la figure 3 est une vue en coupe d'un réacteur chimique ;
la figure 4 est une vue en coupe d'un dispositif d'injection ;
la figure 5 est une vue en perspective d'un cylindre de distribution ;
la figure 6 est une vue en perspective du sélecteur de solvant ;
la figure 7 est une vue du schéma de principe de câblage pour un ensemble doseur volumétrique de solvant-réacteur chimique, et
la figure 8 est une vue du schéma de principe fonctionnel fluidique et pneumatique.

Conformément à l'invention, le multi-synthétiseur de peptides est principalement constitué par des réacteurs chimiques 2 reliés à des doseurs volumétriques de solvant 1 disposés en amont desdits réacteurs chimiques 2 et en aval d'un sélecteur de solvant 4 alimenté en solvant grâce à des bouteilles de solvant 6 sous pression d'azote, par des cylindres de distribution 3 répartissant l'azote, les différents solvants, les vapeurs d'air et d'azote et les déchets vers les différents éléments spécifiques du multi-synthétiseur de peptides, ainsi que par une centrale de réglage et de régulation d'azote très basse pression 5, générant un bullage d'azote dans les réacteurs chimiques 2 et assurant un contact parfait entre le couple résine-amino-acide et les différents solvants, ces moyens étant séquentiellement commandés par un automate programmable 7 ou un micro-ordinateur assurant la mise en œuvre de la technique de synthèse, et le nombre maximum d'ensembles doseur volumétrique de solvant 1-réacteur chimique 2 étant égal au nombre maximum de peptides pouvant être fabriqués simultanément.

Comme le montre la figure 1, le multi-synthétiseur de peptides présente un châssis métallique 8 dont la partie inférieure renferme la centrale de réglage et de régulation d'azote très basse pression 5, ainsi que les bouteilles 6 de solvant sous pression d'azote, dont la partie centrale est constituée par une tablette de travail 9 derrière laquelle se trouve la zone des vannes de sortie des réacteurs chimiques 2 et au-dessus de laquelle est située la zone des réacteurs chimiques 2, et dont la partie supérieure renferme les doseurs volumétriques de solvant 1, derrière lesquels se trouvent les différents cylindres de distribution 3, le sélecteur de solvant 4, ainsi que l'automate programmable 7 ou le micro-ordinateur.

La surface frontale de la partie supérieure présente différents cadrans, dont notamment un cadran illustrant le schéma synoptique du fonctionnement fluidique et pneumatique de l'appareil, un cadran de contrôle et de programmation de l'automate ainsi qu'un cadran de contrôle des anomalies. Les bouteilles 6 de solvant sont disposées sur le fond du châssis métallique 8. Tous les autres organes, en particulier la

centrale de réglage et de régulation 5, les doseurs volumétriques de solvant 1, les réacteurs chimiques 2, les cylindres de distribution 3, le sélecteur de solvant 4 et l'automate programmable 7 ou le micro-ordinateur sont fixés aux différentes parois et aux différents montants du châssis 8 de manière connue, à l'aide de colliers de fixation et de serrage, d'équerres de fixation, de plaques d'assemblage, de tiges et de plaques de serrage, de cavaliers de fixation ainsi que de différents types de vis et d'écrous. L'alimentation en azote sera assurée par une bouteille d'azote 48 à travers un dispositif de détente 47 permettant d'abaisser la pression délivrée par la bouteille d'azote de 200 bar à 15 bar pour alimenter à travers une vanne de contrôle 51 deux ensembles détendeur 46-filtre d'humidité 45 affectés respectivement pour l'alimentation de la centrale de réglage et de régulation 5. La bouteille d'azote 48 est disposée à proximité de l'appareil et reliée à l'un des cylindres de distribution 3. Les ensembles doseur volumétrique de solvant 1-réacteur chimique 2 sont montés en parallèle au moyen de tuyaux 10 reliés entre eux par des raccords union 11, et dans lesquels sont injectés des solvants ou de l'azote, des vannes 12 étant insérées dans les tuyaux 10, par des raccords 11' entre vannes 12 et tuyaux 10, les raccords union 11 et les raccords vanne-tuyau 11' ainsi que les tuyaux 10 étant réalisés en polytétrafluoréthylène (PTFE) ou en éthylène propylène fluoré copolymère (FEP).

Tous les raccords sont de type connu, de préférence de la société Bohlender. Le nombre d'ensembles doseur volumétrique de solvant 1-réacteur chimique 2 peut varier d'un appareil à l'autre. Dans le mode de réalisation décrit, il sera avantageusement de six, c'est-à-dire six doseurs volumétriques de solvant 1 et six réacteurs chimiques 2. Il sera donc possible de fabriquer simultanément six peptides de séquences identiques ou différentes et en quantités différentes. Les derniers peuvent varier d'une centaine de milligrammes à quelques grammes. Chaque peptide sera réalisé dans un ensemble doseur volumétrique de solvant 1-réacteur chimique 2, et ceci de manière totalement indépendante de la fabrication des autres peptides.

Comme le montre la figure 2, chaque doseur volumétrique de solvant 1 est constitué par deux cylindres, à savoir un cylindre principal 13 d'introduction des différents solvants sous pression d'azote et une colonne de mesure 14 de détermination du volume exact de solvant à injecter dans le réacteur chimique 2. Le cylindre principal 13 est traversé par une colonne 15 dont l'orifice supérieur 16 est l'entrée principale des différents solvants sous pression d'azote et fait saillie par rapport à l'extrémité supérieure du cylindre principal 13, et dont l'extrémité inférieure 17 débouche sur un disque 18 relié à la colonne 15 par deux attaches 19 et 20, l'extrémité inférieure du cylindre principal 13 étant pourvue d'un orifice de sortie 21 relié à une colonne 22 située dans le prolongement du cylindre principal 13 pour l'injection de solvant sous pression d'azote dans le réacteur chimique 2. La colonne de mesure 14 présente, d'une part, à son extrémité supérieure, un orifice 23 d'entrée d'azote à haute pression et d'échappement d'air, relié à un dispositif d'évacuation sous faible dépression constitué d'un filtre 49 à charbon actif et d'un extracteur 50, et, d'autre part, près de cette dite extrémité supérieure, une conduite de liaison 24 au cylindre principal 13, son extrémité inférieure étant reliée à la colonne 22. La colonne de mesure 14 est associée à une barrette de détecteurs optoélectroniques 25 déterminant des volumes différents de solvant. Ces détecteurs optoélectroniques sont avantageusement positionnés de manière à sélectionner sept volumes de solvant, à savoir 5 ml, 20 ml, 30 ml, 40 ml, 50 ml, 60 ml et 70 ml. La présélection du volume est réalisée par programmation en fonction de la quantité de produit à fabriquer par le réacteur chimique 2 qui lui est associé. Les doseurs volumétriques de solvant 1 permettent donc d'injecter simultanément différents solvants en quantité différente dans les réacteurs chimiques 2.

Selon la figure 3, chaque réacteur chimique 2 présente quatre orifices, à savoir un orifice 26 situé au sommet du réacteur chimique 2, contenant un dispositif d'injection 27 du solvant sous pression, ainsi que de l'azote sous pression lors du vidage du réacteur chimique 2 de son liquide, et relié par un ensemble raccord en Té-vannes-raccord à la colonne 22 du doseur volumétrique 1, un orifice 28 d'introduction des amino-acides et de la résine permettant également le prélèvement d'échantillons du couple résine-peptide pour le contrôle, aux stades essentiels de la synthèse, un orifice 29 relié à un dispositif d'évacuation sous faible dépression constitué d'un filtre 49 à charbon actif et d'un extracteur 50 pour l'évacuation de l'air ou de l'azote résiduel contenu dans le réacteur chimique 2 lors de son remplissage, les deux orifices 28 et 29 étant situés de part et d'autre de l'orifice 26, et un orifice 30 d'évacuation des solvants et d'admission de l'azote très basse pression pour agitation par bullage, et situé à l'extrémité inférieure du réacteur chimique 2. L'expérimentateur dépose la résine à l'intérieur des différents réacteurs chimiques 2, résine sur laquelle est couplée la chaîne d'amino-acides en croissance. Celle-ci subit alors un traitement de déprotection du groupement protecteur t-butyloxycarbonyle par les différents solvants au moyen de lavages successifs. Puis, après introduction sous pression du solvant, la résine en suspension subit une agitation par bullage d'azote sous faible pression.

Conformément à une autre caractéristique de l'invention, tous les orifices du réacteur chimique 2 sont munis de vannes de contrôle 12 assurant successivement ou simultanément le contrôle des entrées et sorties du réacteur et pouvant totalement isoler ce dernier de son contexte périphérique, l'orifice 30 situé à l'extrémité inférieure du réacteur chimique 2 étant relié, d'une part, à une vanne 131 de déchet et, d'autre part, à une vanne 130 de bullage à l'aide d'un raccord en Té 11".

Chaque réacteur chimique 2, associé à chaque doseur volumétrique de solvant 1, forme ainsi un ensemble qui se comporte en unité indépendante. Il est donc possible de mettre en œuvre un ou plusieurs réacteurs 2, indépendants les uns des autres, d'en isoler partiellement un ou plusieurs au cours du

déroulement de la synthèse, de moduler la quantité de peptides produite par chacun des réacteurs 2, et, par conséquent, de pouvoir synthétiser simultanément des quantités variables de peptides de séquences différentes.

Conformément à la figure 4, le dispositif d'injection 27 du solvant sous pression présente à sa partie supérieure un raccord de couplage 31, et est composé d'un cylindre creux 32, dans lequel est introduit un cylindre plein 33 cannelé, de diamètre légèrement inférieur, et présentant sur sa base inférieure une section élargie en forme de tronc de cône 34 permettant une distribution radiale et laminaire du flux de liquide injecté dans le réacteur chimique 2.

L'angle du tronc de cône 34 est, de préférence, compris entre 50° et 70° et la hauteur h du dispositif d'injection 27 insérée dans le col de l'orifice 26 est prévue telle que le liquide injecté vienne lécher la paroi interne du réacteur chimique 2 ainsi que la paroi interne des orifices 28 et 29.

La hauteur h du dispositif d'injection insérée dans le col de l'orifice 26 est, de préférence, plus courte d'environ 10 mm par rapport à la longueur dudit col. Le diamètre du dispositif d'injection 27 est inférieur au diamètre du col de l'orifice 26 du réacteur chimique 2 d'environ 0,5 mm à 1,5 mm, de telle manière qu'un ménisque se forme entre la paroi du verre et l'extrémité inférieure du cylindre creux 32. Ainsi, le liquide injecté récupère toutes les particules de résine pouvant adhérer sur la paroi interne du réacteur chimique 2 et lave simultanément les orifices 28 et 29 du réacteur chimique 2.

Conformément à la figure 5, chaque cylindre de distribution 3 est pourvu d'un canal de distribution concentrique 35, dont les extrémités 36 et 37 forment l'entrée et la sortie du distributeur, la distribution étant effectuée radialement au moyen de canaux 38 disposés sur différents niveaux.

Selon un mode de réalisation, les canaux 38 sont avantageusement au nombre de huit et sur deux niveaux, à savoir quatre canaux par niveau, et sont disposés à 90° l'un par rapport à l'autre.

Selon une autre caractéristique de l'invention, le nombre de cylindres de distribution 3 est avantageusement de huit, à savoir un cylindre de distribution générale des solvants 3A, un cylindre de distribution générale d'azote 3B, un cylindre de distribution d'azote basse pression 3C vers l'orifice 16 des doseurs volumétriques 1 et d'évacuation d'air et d'azote du même orifice 16, un cylindre de distribution d'azote basse pression 3D vers l'orifice 29 des réacteurs chimiques 2 et d'évacuation d'air et d'azote du même orifice 29, un cylindre de distribution générale d'évacuation 3E des vapeurs d'air et d'azote vers une hotte 44, un cylindre de distribution générale d'évacuation des déchets 3F vers le dispositif de récupération des déchets 42, un cylindre de distribution générale 3G d'azote très basse pression vers l'orifice 30 des réacteurs chimiques 2 et un cylindre de distribution d'azote pour le vidage du réacteur chimique 2 (voir figures 7 et 8).

Conformément à la figure 6, le sélecteur de solvant 4 est traversé par un canal de distribution concentrique 39, dont une extrémité 40 est reliée par des tuyaux 10 et des raccords 11, 11' à l'un des cylindres de distribution 3 et dont l'autre extrémité 41 est reliée par des tuyaux 10 et des raccords 11, 11' à un dispositif de récupération de déchets 42, et en ce que les différents solvants sont amenés au canal de distribution 39 au moyen de canaux 43 à ouverture commandée par des vannes 12 et disposés chacun sur un niveau différent selon le pas d'une hélice. Le sélecteur de solvant 4 est conçu de telle sorte qu'il élimine tous risques de contamination des réacteurs chimiques 2, isolés momentanément par des segments de fluide parasite. En effet, lors de l'injection dans les doseurs volumétriques 1 du solvant le plus agressif, en l'occurrence l'acide trifluoroacétique, ce dernier contamine sur son passage les petits canaux d'injection 43 des autres solvants. Mais les canaux 43 étant disposés chacun sur un niveau différent selon le pas d'une hélice de manière à pouvoir étager les solvants suivant un ordre bien précis, du plus agressif au moins agressif à partir du bas du sélecteur de solvant 4, ils sont bien nettoyés par un mouvement de reflux du liquide vers le dispositif de récupération de déchets 42. On assure ainsi un rinçage parfait du sélecteur de solvant 4.

Les cylindres de distribution 3 ainsi que le sélecteur de solvant 4 sont avantageusement réalisés en PTFE.

L'invention a également pour objet un procédé de production de peptides de synthèse mis en œuvre par le multi-synthétiseur de peptides représenté à la figure 1, procédé consistant à réaliser simultanément et indépendamment la synthèse d'un nombre variable de peptides en quantités différentes, de séquences identiques ou différentes, suivant une technique de synthèse commune consistant, pour chaque cycle, soit à partir d'un couple résine-amino-acide que l'on introduit dans chaque réacteur chimique 2, soit à réaliser un tel couple pour chaque ensemble doseur volumétrique de solvant 1-réacteur chimique 2, puis à déprotéger la fonction N-α aminée de chaque amino-acide en éliminant le groupement protecteur t-butyloxycarbonyle par acidolyse et en neutralisant le groupement aminé obtenu sous forme de sel, à coupler le second amino-acide protégé sur la résine, à déprotéger sa fonction N-α aminée et à répéter ce cycle pour chaque peptide à réaliser, de séquence identique ou différente, un nombre de fois égal au nombre d'acides aminés à fixer dans la résine. Ce nombre d'acides aminés pourra donc être différent d'un réacteur chimique 2 à l'autre. Le premier peptide obtenu sera celui comportant le moins d'acides aminés et le dernier obtenu celui qui en comporte le plus, la durée de fabrication de chaque peptide étant proportionnelle au nombre de cycles nécessaires, donc au nombre d'acides aminés à fixer dans la résine.

La première étape de chaque cycle de la technique de synthèse consiste, soit à introduire un couple résine/amino-acide à travers l'orifice 28 de chaque réacteur chimique 2, soit à le réaliser à l'aide du multi-synthétiseur. Pour la réalisation des couples résine/amino-acide, les étapes suivantes, pour chaque

ensemble doseur volumétrique de solvant 1-réacteur chimique 2, sont effectuées sous le contrôle de l'automate programmable 7 ou du micro-ordinateur :

introduction manuelle de résine à travers l'orifice 28 ;

injection d'un solvant sous pression d'azote, à savoir du dichlorométhane, à travers l'orifice 26 ;

introduction d'azote très basse pression à travers l'orifice 30 pour une agitation par bullage d'environ une minute afin de laver et de faire gonfler la résine par agitation du solvant et de la résine ;

introduction d'azote sous pression à travers l'orifice 26 afin d'évacuer le solvant à travers l'orifice 30 ;

répétition de ces trois dernières opérations jusqu'à obtention d'une résine propre ;

introduction manuelle du groupement protecteur t-butyloxycarbonyle amino-acide à travers l'orifice 28 ;

injection d'un solvant sous pression d'azote, à savoir du dichlorométhane, à travers l'orifice 26 ;

introduction d'azote très basse pression à travers l'orifice 30 pendant environ 4 heures pour une agitation par bullage avec introduction manuelle à plusieurs reprises d'agents de couplage et/ou d'activateurs à travers l'orifice 28 ;

introduction d'azote sous pression par l'orifice 26, afin d'évacuer le solvant à travers l'orifice 30 ;

injection d'un solvant sous pression d'azote, à savoir du dichlorométhane, à travers l'orifice 26 ;

introduction d'azote très basse pression à travers l'orifice 30 pendant environ une minute pour une agitation par bullage afin de laver le couple résine-amino-acide ;

introduction d'azote sous pression à travers l'orifice 26, afin d'évacuer le solvant à travers l'orifice 30 ;

répétition de ces trois dernières opérations jusqu'à obtention d'un couple résine-amino-acides propre.

Il sera alors procédé, pour chaque couple résine-amino-acide obtenu, à la déprotection de la fonction N-α aminée, par élimination du groupement protecteur t-butyloxycarbonyle par acidolyse selon les étapes suivantes, effectuées sous le contrôle de l'automate programmable 7 ou du micro-ordinateur pour chaque ensemble doseur volumétrique de solvant 1-réacteur chimique 2 :

injection sous pression d'azote à travers l'orifice 26 d'un mélange de 35 % de dichlorométhane et de 65 % d'acide trifluoroacétique ;

introduction d'azote très basse pression à travers l'orifice 30 pendant environ une minute, pour une agitation par bullage ;

introduction d'azote sous pression à travers l'orifice 26 afin d'évacuer le mélange dichlorométhane-acide trifluoroacétique à travers l'orifice 30 ;

injection du mélange de dichlorométhane et d'acide trifluoroacétique sous pression d'azote dans des proportions égales à travers l'orifice 26 ;

introduction pendant environ 13 minutes d'azote très basse pression à travers l'orifice 30 pour une agitation par bullage ;

introduction d'azote sous pression à travers l'orifice 26 afin d'évacuer le mélange dichlorométhane-acide trifluoroacétique à travers l'orifice 30 ;

injection d'un solvant sous pression d'azote, à savoir du dichlorométhane, à travers l'orifice 26 ;

introduction d'azote très basse pression à travers l'orifice 30 pendant environ une minute pour une agitation par bullage afin de laver le couple résine-amino-acide déprotégé ;

introduction d'azote sous pression à travers l'orifice 26 afin d'évacuer le solvant à travers l'orifice 30 ;

répétition de ces trois dernières opérations ;

injection d'un solvant sous pression d'azote, à savoir du diméthylformamide à travers l'orifice 26, afin d'éliminer les restes d'acide trifluoroacétique ;

introduction d'azote très basse pression à travers l'orifice 30 pendant quelques secondes pour une agitation par bullage ;

introduction d'azote sous pression à travers l'orifice 26 afin d'évacuer le solvant à travers l'orifice 30 ;

injection d'un solvant, à savoir du dichlorométhane, à travers l'orifice 26 ;

introduction d'azote très basse pression à travers l'orifice 30 pendant environ une minute pour une agitation par bullage ;

introduction d'azote sous pression à travers l'orifice 26 afin d'évacuer le solvant à travers l'orifice 30 ;

injection d'un solvant, à savoir du diméthylformamide à travers l'orifice 26 ;

introduction d'azote très basse pression à travers l'orifice 30 pendant environ une minute pour une agitation par bullage ;

introduction d'azote sous pression à travers l'orifice 26 afin d'évacuer le solvant à travers l'orifice 30 ;

répétition de cette dernière opération.

Pour la neutralisation du groupement aminé obtenu sous forme de sel, les étapes suivantes, pour chaque ensemble doseur volumétrique de solvant 1-réacteur chimique 2 sont réalisées sous le contrôle de l'automate programmable 7 et du micro-ordinateur :

injection sous pression d'azote d'un mélange de 10 % de diisopropyléthylamine et de 90 % de dichlorométhane ou de diméthylformamide à travers l'orifice 26 ;

introduction d'azote très basse pression à travers l'orifice 30 pendant environ une minute pour une agitation par bullage ;

introduction d'azote sous pression à travers l'orifice 26 afin d'évacuer le mélange diisopropyléthylamine-dichlorométhane ou le mélange diisopropyléthylamine-diméthylformamide à travers l'orifice 30 ;

répétition de cette dernière opération ;

injection d'un solvant sous pression d'azote, à savoir du dichlorométhane ou du diméthylformamide, à travers l'orifice 26 ;

introduction d'azote très basse pression à travers l'orifice 30 pendant environ une minute pour une agitation par bullage ;

introduction d'azote sous pression à travers l'orifice 26 afin d'évacuer le solvant à travers l'orifice 30 ;

triple reprise de cette dernière opération pour le lavage du couple résine-amino-acide ;

prélèvement manuel à travers l'orifice 28 d'un échantillon du couple résine-amino-acide pour la vérification de la réaction de déprotection à l'aide d'un test à la ninhydrine.

Enfin, pour coupler le second amino-acide protégé sur le couple résine-amino-acide obtenu, les étapes suivantes, pour chaque ensemble doseur volumétrique de solvant 1-réacteur chimique 2 sont réalisées sous le contrôle de l'automate programmable 7 ou du micro-ordinateur :

introduction manuelle de l'amino-acide protégé, activé ou non, à travers l'orifice 28 ;

injection d'un solvant sous pression d'azote, à savoir du dichlorométhane ou du diméthylformamide, à travers l'orifice 26 ;

introduction d'azote très basse pression à travers l'orifice 30 pendant une durée comprise entre 10 et 60 minutes pour une agitation par bullage avec introduction manuelle d'agents de couplage et/ou d'activateurs à travers l'orifice 28 ;

prélèvement manuel à travers l'orifice 28 d'un échantillon de l'ensemble résine-amino-acide obtenu et test à la ninhydrine ;

soit introduction d'azote sous pression à travers l'orifice 26 afin d'évacuer le solvant à travers l'orifice 30, si le test effectué lors de l'opération précédente est négatif, soit reprise des opérations au début de la neutralisation du groupement aminé obtenu sous forme de sel, si le test effectué lors de ladite opération précédente est positif ;

injection d'un solvant sous pression d'azote, à savoir du dichlorométhane, à travers l'orifice 26 ;

introduction d'azote très basse pression à travers l'orifice 30 pendant environ une minute pour une agitation par bullage afin de laver l'ensemble résine-amino-acide obtenu ;

introduction d'azote sous pression à travers l'orifice 26 afin d'évacuer le solvant à travers l'orifice 30 ;

triple répétition de cette dernière opération jusqu'à obtention d'un ensemble propre.

Pour tout le cycle, la pression d'azote nécessaire à l'injection du solvant est comprise entre 300 et 400 mbar, de préférence, entre 340 et 360 mbar, et la pression d'azote pour l'agitation par bullage est comprise entre 10 et 70 mbar, de préférence, entre 20 et 35 mbar. Cette très basse pression est maintenue constante à $\pm$ 1/10 mbar grâce à la centrale de réglage et de régulation d'azote 5. Le volume de solvant à injecter est compris entre 15 et 25 ml par gramme de résine, de préférence 20 ml.

L'automate programmable 7 ou le micro-ordinateur commande chaque élément physique du multi-synthétiseur, afin de lui faire exécuter les fonctions nécessaires au déroulement de chaque cycle de la technique de synthèse. S'il s'agit d'un automate programmable, son microprocesseur pourra être avantageusement du type 8032 de la Société Intel, la mémoire de programme de 4 K octets ROM et le langage de programmation assembleur MCS 51.

L'automate programmable ou le micro-ordinateur contrôle donc la chronologie des différentes opérations de chaque cycle, ainsi que chaque opération en elle-même.

Les opérations principales, pour chaque cycle, sont la mesure des volumes de solvant, l'injection de solvant dans chaque réacteur chimique 2, l'agitation par bullage et la vidange dans chaque réacteur chimique 2.

Selon une caractéristique de l'invention, la mesure des volumes de solvant à injecter dans chaque réacteur chimique 2 à travers l'orifice 26 s'effectue sous contrôle de l'automate programmable ou du micro-ordinateur 7 de la manière suivante :

ouverture des vannes d'admission 120 du solvant sélectionné et 120' d'admission d'azote sélectionné ;

ouverture de la vanne d'admission 121 de l'orifice 16 du doseur volumétrique de solvant 1 ;

ouverture de la vanne de mise à l'air 122 du cylindre de distribution 3C ;

injection d'un solvant sous pression d'azote à travers l'orifice 16 du doseur volumétrique 1 jusqu'à détection du niveau par la barrette de détecteurs optoélectroniques 25 ;

fermeture de la vanne d'admission 121 de l'orifice 16 du doseur volumétrique de solvant 1 ;

ajustement du volume préétabli pour chaque ensemble doseur volumétrique 1-réacteur chimique 2 ;

fermeture des vannes d'admission 120 et 120' du couple solvant-azote sélectionné.

Selon une autre caractéristique de l'invention, l'injection du solvant dans chaque réacteur chimique 2 s'effectue sous contrôle de l'automate programmable 7 ou du micro-ordinateur de la manière suivante :

ouverture de la vanne d'admission 123 d'azote du cylindre de distribution 3C de manière à mettre sous pression, à travers l'orifice 16 du doseur volumétrique 1, le solvant contenu dans le cylindre principal 13 et dans la colonne de mesure 14 du doseur volumétrique 1 ;

mise en dépression du réacteur chimique 2 par ouverture de la vanne de détente 125 de l'orifice 29 à travers le cylindre de distribution 3D ;

ouverture de la vanne d'admission 126 de l'orifice 26 ;

mise en dépression durant environ 5 secondes du doseur volumétrique 1 par ouverture de la vanne de mise à l'air 122 du cylindre de distribution 3C.

Conformément à une autre caractéristique de l'invention, l'agitation par bullage dans chaque réacteur chimique 2 s'effectue sous contrôle de l'automate programmable 7 ou du micro-ordinateur de la manière suivante :

mise en dépression du réacteur par ouverture de la vanne de détente 125 de l'orifice 29 à travers le cylindre de distribution 3D ;

temporisation de 5 secondes pour la mise en détente du réacteur chimique 2 ;

ouverture des vannes générales 127, 127' de distribution très basse pression d'azote en amont du cylindre de distribution 3G ;

ouverture de la vanne d'admission d'azote 130 de l'orifice 30 ;

ouverture de la vanne 128 contrôlant l'impulsion de surpression durant une demi-seconde ;

fermeture de la vanne 128 ;

distribution d'azote à très basse pression contrôlée pour une agitation par bullage à travers l'orifice 30 ;

fermeture de la vanne 130 d'admission d'azote de l'orifice 30 ;

temporisation de 200 ms avant le début de la vidange.

Selon une autre caractéristique de l'invention, la vidange de chaque réacteur chimique 2 s'effectue sous contrôle de l'automate programmable ou du micro-ordinateur 7 de la manière suivante :

ouverture de la vanne 126' d'admission d'azote de l'orifice 26 du cylindre de distribution 3H ;

ouverture de la vanne de déchet 124 du cylindre de distribution 3F ;

ouverture de la vanne d'évacuation des solvants 131 de l'orifice 30 ;

mise en dépression du réacteur par ouverture de la vanne de détente 125 de l'orifice 29 à travers le cylindre de distribution 3D ;

temporisation de 5 secondes pour la mise en détente du réacteur chimique 2.

Les opérations annexes pour chaque cycle sont le rinçage du sélecteur de solvant 4 et le remplissage des bouteilles de solvant 6. Le rinçage du sélecteur de solvant 4 des segments résiduels du solvant précédent s'effectue sous contrôle de l'automate programmable 7 ou du micro-ordinateur de la manière suivante :

ouverture de la vanne d'admission 123 d'azote du cylindre de distribution 3C ;

ouverture de la vanne-déchet 129 du sélecteur de solvant 4 ;

ouverture de la vanne d'admission 121 de l'orifice 16.

Le remplissage des bouteilles de solvant, quant à lui, s'effectue sous contrôle de l'automate programmable 7 ou du micro-ordinateur de la manière suivante :

fermeture de la vanne basse pression 132 du cylindre de distribution 3B ;

ouverture de la vanne de mise à l'air 133 du cylindre de distribution 3B ;

ouverture de la vanne 120' de la bouteille de solvant considérée ;

remplissage manuel des bouteilles 6 ;

fermeture de la vanne 120' de la bouteille de solvant considérée ;

fermeture de la vanne de mise à l'air 133 du cylindre de distribution 3B ;

ouverture de la vanne basse pression 132 du cylindre de distribution 3B.

Enfin, l'automate programmable ou le micro-ordinateur contrôle également l'affichage par cristaux liquides, l'impression par imprimante, l'injection de solvant pour la compensation de l'évaporation lors des longues périodes d'agitation par bullage, ainsi que les dispositifs d'alarme et de sécurité.

Bien entendu, l'invention n'est pas limitée aux modes de réalisation décrits et représentés aux dessins annexés. Des modifications restent possibles, notamment du point de vue de la constitution des divers éléments, ou par substitution d'équivalents techniques, sans sortir pour autant du domaine de protection de l'invention.

## Revendications

1. Multi-synthétiseur de peptides semi-automatique en phase solide, caractérisé en ce qu'il est principalement constitué par des réacteurs chimiques (2) reliés à des doseurs volumétriques de solvant (1) disposés en amont desdits réacteurs chimiques (2) et en aval d'un sélecteur de solvant (4) alimenté en solvant grâce à des bouteilles de solvant (6) sous pression d'azote, par des cylindres de distribution (3), ainsi que par une centrale de réglage et de régulation d'azote très basse pression (5), ces moyens étant séquentiellement commandés par un automate programmable (7) ou un micro-ordinateur assurant la mise en œuvre de la technique de synthèse, et le nombre maximum d'ensembles doseur volumétrique de solvant (1)-réacteur chimique (2) étant égal au nombre maximum de peptides pouvant être fabriqués simultanément.

2. Multi-synthétiseur de peptides selon la revendication 1, caractérisé en ce qu'il présente un châssis métallique (8) dont la partie inférieure renferme la centrale de réglage et de régulation d'azote très basse pression (5) ainsi que les bouteilles (6) de solvant sous pression d'azote, dont la partie centrale est constituée par une tablette de travail (9) derrière laquelle se trouve la zone des vannes de sortie des réacteurs chimiques (2) et au-dessus de laquelle est située la zone des réacteurs chimiques (2), et dont la

partie supérieure renferme les doseurs volumétriques de solvant (1), derrière lesquels se trouvent les différents cylindres de distribution (3), le sélecteur de solvant (4), ainsi que l'automate programmable (7) ou le micro-ordinateur.

3. Multi-synthétiseur de peptides selon l'une quelconque des revendications 1 et 2, caractérisé en ce que les ensembles doseur volumétrique de solvant (1)-réacteur chimique (2) sont montés en parallèle au moyen de tuyaux (10) reliés entre eux par des raccords union (11), et dans lesquels sont injectés des solvants ou de l'azote, des vannes (12) étant insérées dans les tuyaux (10), par des raccords (11') entre vannes (12) et tuyaux (10), et en ce que les raccords union (11) et les raccords vanne-tuyau (11') ainsi que les tuyaux (10) sont réalisés en polytétrafluoréthylène (PTFE) ou en éthylène propylène fluoré copolymère (FEP).

4. Multi-synthétiseur de peptides selon la revendication 1, caractérisé en ce que chaque doseur volumétrique de solvant (1) est constitué par deux cylindres, à savoir un cylindre principal (13) d'introduction des différents solvants sous pression d'azote et une colonne de mesure (14) de détermination du volume exact de solvant à injecter dans le réacteur chimique (2), le cylindre principal (13) étant traversé par une colonne (15) dont l'orifice supérieur (16) est l'entrée principale des différents solvants sous pression d'azote et fait saillie par rapport à l'extrémité supérieure du cylindre principal (13), et dont l'extrémité inférieure (17) débouche sur un disque (18) relié à la colonne (15) par deux attaches (19) et (20), l'extrémité inférieure du cylindre principal (13) étant pourvue d'un orifice de sortie (21) relié à une colonne (22) située dans le prolongement du cylindre principal (13) pour l'injection de solvant sous pression d'azote dans le réacteur chimique (2), et la colonne de mesure (14) présentant, d'une part, à son extrémité supérieure, un orifice (23) d'entrée d'azote à haute pression et d'échappement d'air, relié à un dispositif d'évacuation sous faible dépression constitué d'un filtre (49) à charbon actif et d'un extracteur (50), et, d'autre part, près de cette dite extrémité supérieure, une conduite de liaison (24) au cylindre principal (13), son extrémité inférieure étant reliée à la colonne (22).

5. Multi-synthétiseur de peptides selon la revendication 1, caractérisé en ce que chaque réacteur chimique (2) présente quatre orifices, à savoir un orifice (26) situé au sommet du réacteur chimique (2), contenant un dispositif d'injection (27) du solvant sous pression ainsi que de l'azote sous pression lors du vidage du réacteur chimique de son liquide, et relié par un ensemble raccord en Té-vannes-raccord à la colonne (22) du doseur volumétrique (1), un orifice (28) d'introduction des aminoacides et de la résine permettant également le prélèvement d'échantillons du couple résine-peptide pour le contrôle, aux stades essentiels de la synthèse, un orifice (29) relié à un dispositif d'évacuation sous faible dépression constitué d'un filtre (49) à charbon actif et d'un extracteur (50) pour l'évacuation de l'air ou de l'azote résiduel contenu dans le réacteur chimique (2) lors de son remplissage, les deux orifices (28) et (29) étant situés de part et d'autre de l'orifice (26), et un orifice (30) d'évacuation des solvants et d'admission de l'azote très basse pression pour agitation par bullage, et situé à l'extrémité inférieure du réacteur chimique (2), tous les orifices du réacteur chimique (2) étant, en outre, munis de vannes de contrôle (12) assurant successivement ou simultanément le contrôle des entrées et sorties du réacteur et pouvant totalement isoler ce dernier de son contexte périphérique, l'orifice (30) situé à l'extrémité inférieure du réacteur chimique (2) étant relié, d'une part, à une vanne (131) de déchet et, d'autre part, à une vanne (130) de bullage à l'aide d'un raccord en Té (11").

6. Multi-synthétiseur de peptides selon la revendication 5, caractérisé en ce que le dispositif d'injection (27) du solvant sous pression présente à sa partie supérieure un raccord de couplage (31), et est composé d'un cylindre creux (32), dans lequel est introduit un cylindre plein (33) cannelé, de diamètre légèrement inférieur, et présentant sur sa base inférieure une section élargie en forme de tronc de cône (34) permettant une distribution radicale et laminaire du flux de liquide injecté dans le réacteur chimique (2).

7. Multi-synthétiseur de peptides selon la revendication 6, caractérisé en ce que l'angle ($\alpha$) du tronc de cône (34) est compris entre 50° et 70° et la hauteur (h) du dispositif d'injection (27) insérée dans le col de l'orifice (26) est plus courte d'environ 10 mm par rapport à la longueur dudit col (26), de telle manière que le liquide injecté vienne lécher la paroi interne du réacteur chimique (2) ainsi que les parois internes des orifices (28) et (29).

8. Multi-synthétiseur de peptides selon la revendication 1, caractérisé en ce que chaque cylindre de distribution (3) est pourvu d'un canal de distribution concentrique (35), dont les extrémités (36) et (37) forment l'entrée et la sortie du distributeur, la distribution étant effectuée radialement au moyen de canaux (38) disposés sur différents niveaux.

9. Multi-synthétiseur de peptides selon la revendication 8, caractérisé en ce que le nombre de cylindres de distribution (3) est avantageusement de huit, à savoir un cylindre de distribution générale des solvants (3A), un cylindre de distribution générale d'azote (3B), un cylindre de distribution d'azote basse pression (3C) vers l'orifice (16) des doseurs volumétriques (1), et d'évacuation d'air et d'azote du même orifice (16), un cylindre de distribution d'azote basse pression (3D) vers l'orifice (29) des réacteurs chimiques (2) et d'évacuation d'air et d'azote du même orifice (29), un cylindre de distribution générale d'évacuation (3E) des vapeurs d'air et d'azote vers une hotte (44), un cylindre de distribution générale d'évacuation des déchets (3F) vers le dispositif de récupération des déchets (42), un cylindre de distribution générale (3G) d'azote très basse pression vers l'orifice (30) des réacteurs chimiques (2) et un cylindre de distribution d'azote pour le vidage du réacteur chimique (2).

10. Multi-synthétiseur de peptides selon la revendication 1, caractérisé en ce que le sélecteur de solvant (4) est traversé par un canal de distribution concentrique (39), dont une extrémité (40) est reliée par des tuyaux (10) et des raccords (11, 11') à l'un des cylindres de distribution (3) et dont l'autre extrémité (41) est reliée par des tuyaux (10) et des raccords (11, 11') à un dispositif de récupération de déchets (42), et en ce que les différents solvants sont amenés au canal de distribution (39) au moyen de canaux (43) à ouverture commandée par des vannes (12) et disposés chacun sur un niveau différent selon le pas d'une hélice.

11. Procédé de production de peptides de synthèse mis en œuvre par le dispositif suivant l'une quelconque des revendications 1 à 10, caractérisé en ce qu'il consiste à réaliser simultanément et indépendamment la synthèse d'un nombre variable de peptides en quantités différentes, de séquences identiques ou différentes, suivant une technique de synthèse commune consistant, pour chaque cycle, soit à partir d'un couple résine-amino-acide que l'on introduit dans chaque réacteur chimique (2), soit à réaliser un tel couple pour chaque ensemble doseur volumétrique de solvant (1)-réacteur chimique (2), puis à déprotéger la fonction N-α aminée de chaque amino-acide en éliminant le groupement protecteur t-butyloxycarbonyle par acidolyse et en neutralisant le groupement aminé obtenu sous forme de sel, à coupler le second amino-acide protégé sur la résine, à déprotéger sa fonction N-α aminée et à répéter ce cycle pour chaque peptide à réaliser, de séquence identique ou différente, un nombre de fois égal au nombre d'acides aminés à fixer dans la résine.

12. Procédé de production de peptides selon la revendication 11, caractérisé en ce que, pour la réalisation des couples résine-amino-acide, les étapes suivantes, pour chaque ensemble doseur volumétrique de solvant (1)-réacteur chimique (2), sont effectuées sous le contrôle de l'automate programmable (7) ou du micro-ordinateur :

introduction manuelle de résine à travers l'orifice (28) ;

injection d'un solvant sous pression d'azote, à savoir du dichlorométhane, à travers l'orifice (26) ;

introduction d'azote très basse pression à travers l'orifice (30) pour une agitation par bullage d'environ une minute afin de laver et de faire gonfler la résine par agitation du solvant et de la résine ;

introduction d'azote sous pression à travers l'orifice (26) afin d'évacuer le solvant à travers l'orifice (30) ;

répétition de ces trois dernières opérations jusqu'à obtention d'une résine propre ;

introduction manuelle du groupement protecteur t-butyloxycarbonyle amino-acide à travers l'orifice (28) ;

injection d'un solvant sous pression d'azote, à savoir du dichlorométhane, à travers l'orifice (26) ;

introduction d'azote très basse pression à travers l'orifice (30) pendant environ 4 heures pour une agitation par bullage avec introduction manuelle à plusieurs reprises d'agents de couplage et/ou d'activateurs à travers l'orifice (28) ;

introduction d'azote sous pression par l'orifice (26), afin d'évacuer le solvant à travers l'orifice (30) ;

injection d'un solvant sous pression d'azote, à savoir du dichlorométhane, à travers l'orifice (26) ;

introduction d'azote très basse pression à travers l'orifice (30) pendant environ une minute pour une agitation par bullage afin de laver le couple résine-amino-acide ;

introduction d'azote sous pression à travers l'orifice (26), afin d'évacuer le solvant à travers l'orifice (30) ;

répétition de ces trois dernières opérations jusqu'à obtention d'un couple résine-amino-acide propre.

13. Procédé de production de peptides selon l'une quelconque des revendications 11 et 12, caractérisé en ce que pour la déprotection de la fonction N-α aminée de l'amino-acide protégé par élimination du groupement protecteur t-butyloxycarbonyle par acidolyse, les étapes suivantes, pour chaque ensemble doseur volumétrique de solvant (1)-réacteur chimique (2), sont effectuées sous le contrôle de l'automate programmable (7) ou du micro-ordinateur :

injection sous pression d'azote à travers l'orifice (26) d'un mélange de 35 % de dichlorométhane et de 65 % d'acide trifluoroacétique ;

introduction d'azote très basse pression à travers l'orifice (30) pendant environ une minute, pour une agitation par bullage ;

introduction d'azote sous pression à travers l'orifice (26) afin d'évacuer le mélange dichlorométhane-acide trifluoroacétique à travers l'orifice (30) ;

injection du mélange de dichlorométhane et d'acide trifluoroacétique sous pression d'azote dans des proportions égales à travers l'orifice (26) ;

introduction pendant environ 13 minutes d'azote très basse pression à travers l'orifice (30) pour une agitation par bullage ;

introduction d'azote sous pression à travers l'orifice (26) afin d'évacuer le mélange dichlorométhane-acide trifluoroacétique à travers l'orifice (30) ;

injection d'un solvant sous pression d'azote, à savoir du dichlorométhane, à travers l'orifice (26) ;

introduction d'azote très basse pression à travers l'orifice (30) pendant environ une minute pour une agitation par bullage afin de laver le couple résine-amino-acide déprotégé ;

introduction d'azote sous pression à travers l'orifice (26) afin d'évacuer le solvant à travers l'orifice (30) ;

répétition de ces trois dernières opérations ;

injection d'un solvant sous pression d'azote, à savoir du diméthylformamide à travers l'orifice (26), afin d'éliminer les restes d'acide trifluoroacétique ;

introduction d'azote très basse pression à travers l'orifice (30) pendant quelques secondes pour une agitation par bullage ;

introduction d'azote sous pression à travers l'orifice (26) afin d'évacuer le solvant à travers l'orifice (30) ;

injection d'un solvant, à savoir du dichlorométhane, à travers l'orifice (26) ;

introduction d'azote très basse pression à travers l'orifice (30) pendant environ une minute pour une agitation par bullage ;

introduction d'azote sous pression à travers l'orifice (26) afin d'évacuer le solvant à travers l'orifice (30) ;

injection d'un solvant, à savoir du diméthylformamide à travers l'orifice (26) ;

introduction d'azote très basse pression à travers l'orifice (30) pendant environ une minute pour une agitation par bullage ;

introduction d'azote sous pression à travers l'orifice (26) afin d'évacuer le solvant à travers l'orifice (30) ;

répétition de cette dernière opération.

14. Procédé de production de peptides selon la revendication 11, caractérisé en ce que pour la neutralisation du groupement aminé obtenu sous forme de sel, les étapes suivantes, pour chaque ensemble doseur volumétrique de solvant (1)-réacteur chimique (2) sont réalisées sous le contrôle de l'automate programmable (7) ou du micro-ordinateur :

injection sous pression d'azote d'un mélange de 10 % de diisopropyléthylamine et de 90 % de dichlorométhane ou de diméthylformamide à travers l'orifice (26) ;

introduction d'azote très basse pression à travers l'orifice (30) pendant environ une minute pour une agitation par bullage ;

introduction d'azote sous pression à travers l'orifice (26) afin d'évacuer le mélange diisopropyléthylamine-dichlorométhane ou le mélange diisopropyléthylamine-diméthylformamide à travers l'orifice (30) ;

répétition de cette dernière opération ;

injection d'un solvant sous pression d'azote, à savoir du dichlorométhane ou du diméthylformamide à travers l'orifice (26) ;

introduction d'azote très basse pression à travers l'orifice (30) pendant environ une minute pour une agitation par bullage ;

introduction d'azote sous pression à travers l'orifice (26) afin d'évacuer le solvant à travers l'orifice (30) ;

triple reprise de cette dernière opération pour le lavage du couple résine-amino-acide ;

prélèvement manuel à travers l'orifice (28) d'un échantillon du couple résine-amino-acide pour la vérification de la réaction de déprotection à l'aide d'un test à la ninhydrine.

15. Procédé de production de peptides selon la revendication 11, caractérisé en ce que pour coupler le second amino-acide protégé sur le couple résine-amino-acide obtenu, les étapes suivantes, pour chaque ensemble doseur volumétrique de solvant (1)-réacteur chimique (2) sont réalisées sous le contrôle de l'automate programmable (7) ou du micro-ordinateur :

introduction manuelle de l'amino-acide protégé, activé ou non, à travers l'orifice 28 ;

injection d'un solvant sous pression d'azote, à savoir du dichlorométhane ou du diméthylformamide à travers l'orifice (26) ;

introduction d'azote très basse pression à travers l'orifice (30) pendant une durée comprise entre 10 et 60 minutes pour une agitation par bullage avec introduction manuelle d'agents de couplage et/ou d'activateurs à travers l'orifice (28) ;

prélèvement manuel à travers l'orifice (28) d'un échantillon de l'ensemble résine-amino-acide obtenu et test à la ninhydrine ;

soit introduction d'azote sous pression à travers l'orifice (26) afin d'évacuer le solvant à travers l'orifice (30) si le test effectué lors de l'opération précédente est négatif, soit reprise des opérations au début de la neutralisation du groupement aminé obtenu sous forme de sel, si le test effectué lors de ladite opération précédente est positif ;

injection d'un solvant sous pression d'azote, à savoir du dichlorométhane, à travers l'orifice (26) ;

introduction d'azote très basse pression à travers l'orifice (30) pendant environ une minute pour une agitation par bullage afin de laver l'ensemble résine-amino-acide obtenu ;

introduction d'azote sous pression à travers l'orifice (26) afin d'évacuer le solvant à travers l'orifice (30) ;

triple répétition de cette dernière opération jusqu'à obtention d'un ensemble propre.

16. Procédé de production de peptides selon l'une quelconque des revendications 11 à 15, caractérisé en ce que la mesure des volumes de solvant à injecter dans chaque réacteur chimique (2) à travers l'orifice (26) s'effectue sous contrôle de l'automate programmable (7) ou du micro-ordinateur de la manière suivante :

ouverture des vannes d'admission (120) du solvant sélectionné et (120') d'admission d'azote sélectionné ;

ouverture de la vanne d'admission (121) de l'orifice (16) du doseur volumétrique de solvant (1) ;

ouverture de la vanne de mise à l'air (122) du cylindre de distribution (3C) ;

injection d'un solvant sous pression d'azote à travers l'orifice (16) du doseur volumétrique (1) jusqu'à détection du niveau par la barrette de détecteurs optoélectroniques (25) ;

fermeture de la vanne d'admission (121) de l'orifice (16) du doseur volumétrique de solvant (1) ;

ajustement du volume préétabli pour chaque ensemble doseur volumétrique (1)-réacteur chimique (2) ;

fermeture des vannes d'admission (120) et (120') du couple solvant-azote sélectionné.

17. Procédé de production de peptides selon l'une quelconque des revendications 11 à 15, caractérisé en ce que l'injection du solvant dans chaque réacteur chimique (2) s'effectue sous contrôle de l'automate programmable (7) ou du micro-ordinateur de la manière suivante :

ouverture de la vanne d'admission (123) d'azote du cylindre de distribution (3C) de manière à mettre sous pression, à travers l'orifice (16) du doseur volumétrique (1), le solvant contenu dans le cylindre principal (13) et dans la colonne de mesure (14) du doseur volumétrique (1) ;

mise en dépression du réacteur chimique (2) par ouverture de la vanne de détente (125) de l'orifice (29) à travers le cylindre de distribution (3D) ;

ouverture de la vanne d'admission (126) de l'orifice (26) ;

mise en dépression durant environ 5 secondes du doseur volumétrique (1) par ouverture de la vanne de mise à l'air (122) du cylindre de distribution (3C).

18. Procédé de production de peptides selon l'une quelconque des revendications 11 à 15, caractérisé en ce que l'agitation par bullage dans chaque réacteur chimique (2) s'effectue sous contrôle de l'automate programmable (7) ou du micro-ordinateur de la manière suivante :

mise en dépression du réacteur par ouverture de la vanne de détente (125) de l'orifice (29) à travers le cylindre de distribution (3D) ;

temporisation de 5 secondes pour la mise en détente du réacteur chimique (2) ;

ouverture des vannes générales (127, 127') de distribution très basse pression d'azote en amont du cylindre de distribution (3G) ;

ouverture de la vanne d'admission d'azote (130) de l'orifice (30) ;

ouverture de la vanne (128) contrôlant l'impulsion de surpression durant une demi-seconde ;

fermeture de la vanne (128) ;

distribution d'azote à très basse pression contrôlée pour une agitation par bullage à travers l'orifice (30) ;

fermeture de la vanne (130) d'admission d'azote de l'orifice (30) ;

temporisation de 200 ms avant le début de la vidange.

19. Procédé de production de peptides selon l'une quelconque des revendications 11 à 15, caractérisé en ce que la vidange dans chaque réacteur chimique (2) s'effectue sous contrôle de l'automate programmable (7) ou du micro-ordinateur de la manière suivante :

ouverture de la vanne (126') d'admission d'azote de l'orifice (26) du cylindre de distribution (3H) ;

ouverture de la vanne de déchet (124) du cylindre de distribution (3F) ;

ouverture de la vanne d'évacuation des solvants (131) de l'orifice (30) ;

mise en dépression du réacteur par ouverture de la vanne de détente (125) de l'orifice (29) à travers le cylindre de distribution (3D) ;

temporisation de 5 secondes pour la mise en détente du réacteur chimique (2).

20. Procédé de production de peptides selon l'une quelconque des revendications 11 à 15, caractérisé en ce que le rinçage du sélecteur de solvant (4) des segments résiduels du solvant précédent s'effectue sous contrôle de l'automate programmable (7) ou du micro-ordinateur de la manière suivante :

ouverture de la vanne d'admission (123) d'azote du cylindre de distribution (3C) ;

ouverture de la vanne-déchet (129) du sélecteur de solvant (4) ;

ouverture de la vanne d'admission (121) de l'orifice (16).

21. Procédé de production de peptides selon l'une quelconque des revendications 11 à 15, caractérisé en ce que le remplissage des bouteilles de solvant (6) s'effectue sous contrôle de l'automate programmable (7) ou du micro-ordinateur de la manière suivante :

fermeture de la vanne basse pression (132) du cylindre de distribution (3B) ;

ouverture de la vanne de mise à l'air (133) du cylindre de distribution (3B) ;

ouverture de la vanne (120') de la bouteille de solvant considérée ;

remplissage manuel des bouteilles (6) ;

fermeture de la vanne (120') de la bouteille de solvant considérée ;

fermeture de la vanne de mise à l'air (133) du cylindre de distribution (3B) ;

ouverture de la vanne basse pression (132) du cylindre de distribution (3B).

## Claims

1. A solid phase, semi-automatic peptides multisynthesizer, characterised in that it is made up mainly of chemical reactors (2) connected to volumetric solvent feeders (1) arranged upstream of said chemical

reactors (2) and downstream of a solvent selector (4) supplied with solvent by means of bottles of solvent (6) under nitrogen pressure, of distributing cylinders (3), and of a very low pressure nitrogen control and regulating station (5), these means being sequentially controlled by a programmable automaton (7) or a microcomputer for carrying out the technique of synthesis, and the maximum number of units consisting of volumetric solvent feeder (1) and chemical reactor (2) being equal to the maximum number of peptides which can be produced simultaneously.

2. A peptides multi-synthesizer according to Claim 1, characterised in that it has a metal frame (8), the lower part of which encloses the very low pressure nitrogen control and regulating station (5) as well as bottles (6) of solvent under nitrogen pressure, the central part of which is formed by a work shelf (9) behind which there is located the region of the outlet valves of the chemical reactors (2) and above which there is located the region of the chemical reactors (2), and the upper part of which encloses the volumetric solvent feeders (1), behind which there are located the various distributing cylinders (3), the solvent selector (4) as well as the programmable automaton (7) or the microcomputer.

3. A peptides multi-synthesizer according to any of Claims 1 and 2, characterised in that the units consisting of volumetric solvent feeder (1) and chemical reactor (2) are mounted in parallel by means of pipes (10) which are interconnected by union fittings (11) and in which solvents or nitrogen are injected, valves (12) being inserted into the pipes (10), by fittings (11') between valves (12) and pipes (10), and in that the union fittings (11) and the valve/pipe fittings (11') as well as the pipes (10) are produced from polytetrafluoroethylene (PTFE) or from fluorinated ethene propene copolymer (FEP).

4. A peptides multi-synthesizer according to Claim 1, characterised in that each volumetric solvent feeder (1) is made up of two cylinders, that is a main cylinder (13) for introducing the various solvents under nitrogen pressure and a measuring column (14) for determining the exact volume of solvent to be injected into the chemical reactor (2), the main cylinder (13) being traversed by a column (15), the upper orifice (16) of which is the main inlet for the various solvents under nitrogen pressure and protrudes beyond the upper end of the main cylinder (13), and the lower end (17) of which opens onto a disc (18) connected to the column (15) by two fasteners (19) and (20), the lower end of the main cylinder (13) being provided with an outlet orifice (21) connected to a column (22) located in the extension of the main cylinder (13) for injecting the solvent under nitrogen pressure into the chemical reactor (2), and the measuring column (14) having, on the one hand at its upper end, an orifice (23) for the admission of high pressure nitrogen and for the escape of air, which is connected to an evacuation device which is subjected to a slight depression and is formed by an activated carbon type filter (49) and an extractor (50) and, on the other hand close to said upper end, a conduit (24) for connection to the main cylinder (13), its lower end being connected to the column (22).

5. A peptides multi-synthesizer according to Claim 1, characterised in that each chemical reactor (2) has four orifices, that is an orifice (26) which is located at the top of the chemical reactor (2), contains a device (27) for injecting solvent under pressure as well as nitrogen under pressure as the liquid is emptied from the chemical reactor and connected by an assembly of tee piece-valves-connection to the column (22) of the volumetric feeder (1), an orifice (28) for introducing amino-acids and resin and also allowing the resin-peptide pair to be sampled for monitoring purposes at the crucial stages of synthesis, an orifice (29) connected to an evacuation device which is subjected to a slight depression and is formed by an activated carbon type filter (49) and an extractor (50) for evacuating the air or residual nitrogen contained in the chemical reactor (2) as it is being filled, the two orifices (28) and (29) being located on either side of the orifice (26) and an orifice (30) for evacuation of the solvents and admission of the very low pressure nitrogen for stirring by bubbling, which is located at the lower end of the chemical reactor (2), all the orifices of the chemical reactor (2) also being provided with control valves (12) which ensure that the inlets and outlets of the reactor are monitored successively or simultaneously and can completely isolate the reactor from its surroundings, the orifice (30) located at the lower end of the chemical reactor (2) being connected, on the one hand, to a waste valve (131) and, on the other hand, to a valve (130) for bubbling by means of a tee piece (11").

6. A peptides multi-synthesizer according to Claim 5, characterised in that the device (27) for injecting the solvent under pressure has a coupling fitting (31) at its top and is composed of a hollow cylinder (32) in which there is introduced a grooved solid cylinder (33) of slightly smaller diameter which has, at its base, a widened truncated cone-shaped cross section (34) allowing radial and laminar distribution of the flow of liquid injected into the chemical reactor (2).

7. A peptides multi-synthesizer according to Claim 6, characterised in that the angle ($\alpha$) of the truncated cone (34) is between 50° and 70° and the height (h) of the injection device (27) inserted into the neck of the orifice (26) is about 10 mm shorter than the length of said neck (26) so that the injected liquid licks the internal wall of the chemical reactor (2) as well as the internal walls of the orifices (28) and (29).

8. A peptides multi-synthesizer according to Claim 1, characterised in that each distributing cylinder (3) is provided with a concentric distributing duct (35), the ends (36) and (37) of which form the inlet and outlet of the distributor, distribution being carried out radially by means of ducts (38) arranged at different levels.

9. A peptides multi-synthesizer according to Claim 8, characterised in that there are advantageously eight distributing cylinders (3), that is a general solvent distributing cylinder (3A), a general nitrogen distributing cylinder (3B), a cylinder (3C) for distributing low pressure nitrogen toward the orifice (16) of

the volumetric feeders (1) and for evacuating air and nitrogen from said orifice (16), a cylinder (3D) for distributing low pressure nitrogen toward the orifice (29) of the chemical reactors (2) and for evacuating air and nitrogen from said orifice (29), a general distributing cylinder (3E) for evacuating air and nitrogen vapours toward a fume cupboard (44), a general distributing cylinder (3F) for evacuating waste toward the waste recovery device (42), a cylinder (3G) for the general distribution of very low pressure nitrogen toward the orifice (30) of the chemical reactors (2) and a nitrogen distributing cylinder (2) for emptying the chemical reactor.

10. A peptides multi-synthesizer according to Claim 1, characterised in that the solvent selector (4) is traversed by a concentric distributing duct (39), of which one end (40) is connected by pipes (10) and fittings (11, 11') to one of the distributing cylinders (3) and of which the other end (41) is connected by pipes (10) and fittings (11, 11') to a waste recovery device (42), and in that the various solvents are brought to the distributing duct (39) by means of ducts (43) of which the opening is controlled by valves (12) and which are each arranged at a different level along a screw thread.

11. A process for preparing synthetic peptides carried out using the device according to any one of Claims 1 to 10, characterised in that it involves simultaneously and independently synthesizing a variable number of peptides in different quantities, of identical or different sequences, by a common technique of synthesis involving, for each cycle, either starting from a resin/amino-acid pair which is introduced into each chemical reactor (2), or producing such a pair for each unit consisting of volumetric solvent feeder (1) and chemical reactor (2), then deprotecting the N-α amino group of each amino-acid by eliminating the protective t-butyloxycarbonyl grouping by acidolysis and neutralising the aminated group obtained in the form of a salt, coupling the second protected amino-acid to the resin, deprotecting its N-α amino group and repeating this cycle for each peptide to be produced, whether of identical or different sequence, a number of times equal to the number of aminated acids to be fixed in the resin.

12. A process for preparing peptides according to Claim 11, characterised in that, for producing resin/amino-acid pairs, the following stages, for each unit consisting of volumetric solvent feeder (1) and chemical reactor (2), are carried out under the control of the programmable automaton (7) or the microcomputer :

manual introduction of resin through the orifice (28) ;

injection of a solvent under nitrogen pressure, i. e. dichloromethane, through the orifice (26) ;

introduction of very low pressure nitrogen through the orifice (30) for stirring by bubbling for about one minute so as to wash and expand the resin by stirring the solvent and the resin ;

introduction of nitrogen under pressure through the orifice (26) in order to evacuate the solvent through the orifice (30) ;

repetition of these last three operations until a clean resin is obtained ;

manual introduction of the protective t-butyloxycarbonyl amino-acid group through the orifice (28) ;

injection of a solvent under nitrogen pressure, that is dichloromethane, through the orifice (26) ;

introduction of very low pressure nitrogen through the orifice (30) for about four hours for stirring by bubbling with repeated manual introduction of coupling agents and/or of activators through the orifice (28) ;

introduction of nitrogen under pressure through the orifice (26) in order to evacuate the solvent through the orifice (30) ;

injection of a solvent under nitrogen pressure, that is dichloromethane, through the orifice (26) ;

introduction of very low pressure nitrogen through the orifice (30) for about one minute for stirring by bubbling in order to wash the resin/amino-acid pair ;

introduction of nitrogen under pressure through the orifice (26) in order to evacuate the solvent through the orifice (30) ;

repetition of these last three operations until a clean resin/amino-acid pair is obtained.

13. A process for preparing peptides according to any one of Claims 11 and 12, characterised in that, for deprotection of the N-α amino group of the protected amino-acid by elimination of the protective t-butyloxycarbonyl group by acidolysis, the following stages, for each unit consisting of volumetric solvent feeder (1) and chemical reactor (2), are carried out under the control of the programmable automaton (7) or the microcomputer :

injection under nitrogen pressure through the orifice (26) of a mixture of 35 % of dichloromethane and 65 % of trifluoroacetic acid ;

introduction of very low pressure nitrogen through the orifice (30) for about one minute for stirring by bubbling ;

introduction of nitrogen under pressure through the orifice (26) in order to evacuate the mixture of dichloromethane and trifluoroacetic acid through the orifice (30) ;

injection of the mixture of dichloromethane and trifluoroacetic acid under nitrogen pressure in equal proportions through the orifice (26) ;

introduction for about thirteen minutes of very low pressure nitrogen through the orifice (30) for stirring by bubbling ;

introduction of nitrogen under pressure through the orifice (26) in order to evacuate the mixture of dichloromethane and trifluoroacetic acid through the orifice (30) ;

injection of a solvent under nitrogen pressure, that is dichloromethane, through the orifice (26) ;

introduction of very low pressure nitrogen through the orifice (30) for about one minute for stirring by bubbling in order to wash the resin/deprotected amino-acid pair ;

introduction of nitrogen under pressure through the orifice (26) in order to evacuate the solvent through the orifice (30) ;

repetition of these last three operations ;

injection of a solvent under nitrogen pressure, that is dimethylformamide, through the orifice (26) in order to eliminate the residues of trifluoroacetic acid ;

introduction of very low pressure nitrogen through the orifice (30) for a few seconds for stirring by bubbling ;

introduction of nitrogen under pressure through the orifice (26) in order to evacuate the solvent through the orifice (30) ;

injection of a solvent, that is dichloromethane, through the orifice (26) ;

introduction of very low pressure nitrogen through the orifice (30) for about one minute for stirring by bubbling ;

introduction of nitrogen under pressure through the orifice (26) in order to evacuate the solvent through the orifice (30) ;

injection of a solvent, that is dimethylformamide through the orifice (26) ;

introduction of very low pressure nitrogen through the orifice (30) for about one minute for stirring by bubbling ;

introduction of nitrogen under pressure through the orifice (26) in order to evacuate the solvent through the orifice (30) ;

repetition of this last operation.

14. A process for preparing peptides according to Claim 11, characterised in that, to neutralise the aminated group obtained in the form of a salt, the following stages, for each unit consisting of volumetric solvent feeder (1) and chemical reactor (2), are carried out under the control of the programmable automaton (7) or the microcomputer :

injection under nitrogen pressure of a mixture of 10 % of diisopropylethylamine and 90 % of dichloromethane or dimethylformamide through the orifice (26) ;

introduction of very low pressure nitrogen through the orifice (30) for about one minute for stirring by bubbling ;

introduction of nitrogen under pressure through the orifice (26) in order to evacuate the mixture of diisopropylethylamine and dichloromethane or the mixture of diisopropylethylamine and dimethylformamide through the orifice (30) ;

repetition of this last operation ;

injection of a solvent under nitrogen pressure, that is dichloromethane or dimethylformamide through the orifice (26) ;

introduction of very low pressure nitrogen through the orifice (30) for about one minute for stirring by bubbling ;

introduction of nitrogen under pressure through the orifice (26) in order to evacuate the solvent through the orifice (30) ;

three repetitions of this last operation to wash the resin/amino-acid pair ;

manual sampling through the orifice (28) of the resin/amino-acid pair to check the deprotection reaction by means of a ninhydrin test.

15. A process for preparing peptides according to Claim 11, characterised in that, to couple the second protected amino-acid onto the resin/amino-acid pair obtained, the following stages, for each unit consisting of volumetric solvent feeder (1) and chemical reactor (2), are carried out under the control of the programmable automaton (7) or the microcomputer :

manual introduction of the protected amino-acid, whether or not activated, through the orifice (28) ;

injection of a solvent under nitrogen pressure, that is dichloromethane or dimethylformamide, through the orifice (26) ;

introduction of very low pressure nitrogen through the orifice (30) for a duration of between 10 and 60 minutes for stirring by bubbling with manual introduction of coupling agents and/or activators through the orifice (28) ;

manual sampling through the orifice (28) of the resin/amino-acid assembly obtained and ninhydrin test ;

either introduction of nitrogen under pressure through the orifice (26) in order to evacuate solvent through the orifice (30) if the test carried out during the preceding operation is negative or repetition of the operations at the beginning of neutralisation of the aminated group obtained in the form of a salt, if the test carried out during said preceding operation is positive ;

injection of a solvent under nitrogen pressure, that is dichloromethane, through the orifice (26) ;

introduction of very low pressure nitrogen through the orifice (30) for about one minute for stirring by bubbling in order to wash the resin/amino-acid assembly obtained ;

introduction of nitrogen under pressure through the orifice (26) in order to evacuate the solvent through the orifice (30) ;

three repetitions of the last operation until a clean assembly is obtained.

16. A process for preparing peptides according to any one of Claims 11 to 15, characterised in that the volumes of solvent to be injected into each chemical reactor (2) through the orifice (26) are measured under the control of the programmable automaton (7) or the microcomputer in the following manner:

opening of the valve (120) for admitting the selected solvent and the valve (120') for admitting selected nitrogen;

opening of the inlet valve (121) of the orifice (16) of the volumetric solvent feeder (1);

opening of the venting valve (122) of the distributing cylinder (3C);

injection of a solvent under nitrogen pressure through the orifice (16) of the volumetric feeder (1) until the level is detected by the bar of optoelectronic detectors (25);

closure of the inlet valve (121) of the orifice (16) of the volumetric solvent feeder (1);

adjustment of the predetermined volume for each unit of volumetric feeder (1) and chemical reactor (2);

closure of the inlet valves (120) and (120') of the selected solvent/nitrogen pair.

17. A process for preparing peptides according to any one of Claims 11 to 15, characterised in that the solvent is injected into each chemical reactor (2) under the control of the programmable automaton (7) or of the microcomputer in the following manner;

opening of the nitrogen intake valve (123) of the distributing cylinder (3C) in order to put the solvent contained in the main cylinder (13) and in the measuring column (14) of the volumetric feeder (1) under pressure through the orifice (16) of the volumetric feeder (1);

reducing the pressure of the chemical reactor (2) by opening the release valve (125) of the orifice (29) through the distributing cylinder (3D);

opening the inlet valve (126) of the orifice (26);

reducing the pressure of the volumetric feeder (1) for about 5 seconds by opening the venting valve (122) of the distributing cylinder (3C).

18. A process for preparing peptides according to any one of Claims 11 to 15, characterised in that stirring by bubbling in each chemical reactor (2) is carried out under the control of the programmable automaton (7) or of the microcomputer in the following manner:

reducing the pressure of the reactor by opening the release valve (125) of the orifice (29) through the distributing cylinder (3D);

delay of 5 seconds for decreasing the pressure of the chemical reactor (2);

opening of the general, very low pressure nitrogen distributing valves (127), (127') upstream of the distributing cylinder (3G);

opening of the nitrogen intake valve (130) of the orifice (30);

opening of the valve (128) controlling the pulse of excess pressure lasting half a second;

closure of the valve (128);

distribution of nitrogen at very low controlled pressure for stirring by bubbling through the orifice (30);

closure of the nitrogen intake valve (130) of the orifice (30);

delay of 200 ms before the beginning of drainage.

19. A process for preparing peptides according to any one of Claims 11 to 15, characterised in that drainage into each chemical reactor (2) is carried out under the control of the programmable automaton (7) or of the microcomputer in the following manner;

opening of the nitrogen intake valve (126') of the orifice (26) of the distributing cylinder (3H);

opening of the waste valve (124) of the distributing cylinder (3F);

opening of the solvent evacuating valve (131) of the orifice (30);

reduction of pressure of the reactor by opening the release valve (125) of the orifice (29) through the distributing cylinder (3D);

delay of 5 seconds for decreasing the pressure of the chemical reactor (2).

20. A process for preparing peptides according to any one of Claims 11 to 15, characterised in that the solvent selector (4) is rinsed of residual segments of the preceding solvent under the control of the programmable automaton (7) or of the microcomputer in the following manner:

opening of the nitrogen intake valve (123) of the distributing cylinder (3C);

opening of the waste valve (129) of the solvent selector (4);

opening of the inlet valve (121) of the orifice (16).

21. A process for preparing peptides according to any one of Claims 11 to 15, characterised in that the bottles of solvent (6) are filled under the control of the programmable automaton (7) or of the microcomputer in the following manner:

closure of the low pressure valve (132) of the distributing cylinder (3B);

opening of the venting valve (133) of the distributing cylinder (3B);

opening of the valve (120') of the bottle of solvent under consideration;

manual filling of the bottles (6);

closure of the valve (120') of the bottle of solvent under consideration;

closure of the venting valve (133) of the distributing cylinder (3B);

opening of the low pressure valve (132) of the distributing cylinder (3B).

## Patentansprüche

1. Halbautomatischer Multisynthetisierer für die Herstellung von Peptiden in der festen Phase, dadurch gekennzeichnet, daß er im wesentlichen aus chemischen Reaktoren (2), die an volumetrische Lösungsmitteldosierer (1) angeschlossen sind, welche oberhalb der chemischen Reaktoren (2) und unterhalb eines mit Hilfe von stickstoffgefüllten Lösungsmittelflaschen (6) mit Lösungsmittel gespeisten Lösungsmittelselektors (4) angeordnet sind, aus Verteilerzylindern (3) sowie aus einer Anlage für die Steuerung und Regelung von Stickstoff (5) unter sehr niedrigem Druck besteht, wobei diese Mittel sequentiell durch einen die Synthesetechnik durchführenden, programmierbaren Automaten (7) oder einen Mikrocomputer gesteuert werden, und die maximale Anzahl der Einheiten der volumetrischen Lösungsmitteldosierer (1)-chemischen Reaktoren (2) gleich der maximalen Anzahl der gleichzeitig herstellbaren Anzahl der Peptide ist.

2. Multisynthetisierer für die Herstellung von Peptiden nach Anspruch 1, dadurch gekennzeichnet, daß er ein Metallgehäuse (8) aufweist, in dessen Unterteil die Anlage zur Steuerung und Regelung des Stickstoffs (5) unter sehr niedrigem Druck sowie die stickstoffgefüllten Lösungsmittelflaschen (6) untergebracht sind, dessen Mittelteil aus einer Arbeitsplatte (9) besteht, hinter welcher sich der Bereich für die Auslaßventile der chemischen Reaktoren (2) und über welcher sich der Bereich für die chemischen Reaktoren (2) befindet, dessen Oberteil die volumetrischen Lösungsmitteldosierer (1) aufnimmt, hinter welchen sich die verschiedenen Verteilerzylinder (3), der Lösungsmittelselektor (4) sowie der programmierbare Automat (7) oder der Mikrocomputer befinden.

3. Multisynthetisierer für die Herstellung von Peptiden nach einem der Ansprüche 1 und 2, dadurch gekennzeichnet, daß die Einheit volumetrischer Lösungsmitteldosierer (1)-chemischer Reaktor (2) mit Hilfe von durch Schlauchverbindungsstücke (11) miteinander verbundenen Schläuchen (10) parallel montiert sind, und in welche Lösungsmittel oder Stickstoff eingespritzt wird, wobei in die Schläuche (10) Ventile (12), mittels Verbindungsstücken (11') zwischen den Ventilen (12) und den Schläuchen (10), eingesetzt sind, und daß die Schlauchverbindungsstücke (11) und die Ventil-Schlauch-Verbindungsstücke sowie die Schläuche (10) aus Polytetrafluorethylen (PTFE) oder Fluoräthylenpropylen Copolymer (FEP) hergestellt sind.

4. Multisynthetisierer für die Herstellung von Peptiden nach Anspruch 1, dadurch gekennzeichnet, daß jeder volumetrische Lösungsmitteldosierer (1) aus zwei Zylindern, d. h. einem Hauptzylinder (13) zur Einfüllung der verschiedenen, stickstoffgefüllten Lösungsmittel und einer Meßsäule (14) zur Bestimmung des exakten Volumens des in den chemischen Reaktor (2) einzuspritzenden Lösungsmittels besteht, wobei der Hauptzylinder (13) von einer Säule (15) durchquert wird, deren obere Öffnung (16) den Haupteinlaß für die verschiedenen, stickstoffgefüllten Lösungsmittel bildet und über das obere Ende des Hauptzylinders (13) vorsteht, und dessen unteres Ende (17) an einer Platine (18) mündet, die mit der Säule (15) mittels zweier Verbindungselemente (19) und (20) verbunden ist, wobei das untere Ende des Hauptzylinders (13) mit einer Austrittsöffnung (21) versehen ist, die mit einer in der Verlängerung des Hauptzylinders (13) für die Einspritzung des stickstoffgefüllten Lösungsmittels in den chemische Reaktor (2) sitzenden Säule (22) verbunden ist, und die Meßsäule (14), einerseits, an ihrem oberen Ende eine Öffnung (23) für den Eintritt von Stickstoff unter Hochdruck und den Luftabzug, die mit einer aus einem Aktivkohlefilter (49) und einem Exhaustor (50) bestehenden Ablaßvorrichtung unter schwachem Unterdruck verbunden ist, und andererseits, in der Nähe dieses oberen Endes, eine Verbindungsleitung (24) zu dem Hauptzylinder (13), wobei das untere Ende mit der Säule (22) verbunden ist, aufweist.

5. Multisynthetisierer für die Herstellung von Peptiden nach Anspruch 1, dadurch gekennzeichnet, daß jeder chemische Reaktor (2) vier Öffnungen, nämlich eine an der Spitze des chemischen Reaktors (2) sitzende Öffnung (26), die eine Einspritzvorrichtung (27) für das unter Druck stehende Lösungsmittel sowie für den unter Druck stehenden Stickstoff bei der Entleerung der Flüssigkeit aus dem chemischen Reaktor enthält, und über einen Satz T-Abzweigstück-Ventile-Anschlußstück mit der Säule (22) des volumetrischen Dosierers (1) verbunden ist, eine Öffnung (28) zur Einfüllung der Aminosäuren und des Harzes, die auch Probenentnahmen der Harz-Peptidenverbindung zur Kontrolle in den wesentlichen Synthesestadien ermöglicht, eine Öffnung (29), die mit einer aus einem Aktivkohlefilter (49) und einem Exhaustor (50) für den Luftablaß oder den Ablaß des in dem chemischen Reaktor während des Füllens enthaltenen Rest-Stickstoff bestehenden unter leichtem Unterdruck stehenden Ablaßvorrichtung verbunden ist, wobei die beiden Öffnungen (28) und (29) zu beiden Seiten der Öffnung (26) sitzen, und eine Öffnung (30) für den Ablaß des Lösungsmittels und den Zustrom des unter sehr geringem Druck stehenden Stickstoffs für das Bewegen mittels Durchperlen, am unteren Ende des chemischen Reaktors (2) sitzt, wobei alle Öffnungen des chemischen Reaktors (2) außerdem mit Steuerventilen (12) versehen sind, die nacheinander oder gleichzeitig die Steuerung der Eingänge und Ausgänge des Reaktors gewährleisten und diesen letzteren vollständig von der Verbindung mit seinen peripherischen Geräten isolieren können, wobei die an dem unteren Ende des chemischen Reaktors (2) sitzende Öffnung (30) einerseits mit einem Ventil (131) für die Abfallprodukte und andererseits mit einem Ventil (130) für das Bewegen mittels Durchperlen mit Hilfe eines T-Abzweigstücks (11") verbunden ist.

6. Multisynthetisierer für die Herstellung von Peptiden nach Anspruch 5, dadurch gekennzeichnet, daß die Vorrichtung für das Einspritzen (27) des Lösungsmittels unter Druck an ihrem Oberteil eine Kuppelverbindung (31) aufweist und aus einem Hohlzylinder (32) besteht, in welchen ein geriffelter

Vollzylinder (33) mit etwas kleinerem Durchmesser eingeführt wird, und an seinem Boden einen in Kegelstumpfform (34) erweiteren Querschnitt aufweist, der eine radiale und laminare Verteilung des in des chemischen Reaktor (2) eingespritzten Flüssigkeitsstroms ermöglicht.

7. Multisynthetisierer für die Herstellung von Peptiden nach Anspruch 6, dadurch gekennzeichnet, daß der Winkel (α) des Kegelstumpfes (34) zwischen 50° und 70° liegt und die Höhe (h) der in den Hals der Öffnung (26) eingesetzten Einspritzvorrichtung (27) um etwa 10 mm bezüglich der Länge der Halses (26) kürzer ist, so daß die eingespritzte Flüssigkeit die Innenwand des chemischen Reaktors (2) sowie die Innenwände der Öffnungen (28) und (29) benetzt.

8. Multisynthetisierer für die Herstellung von Peptiden nach Anspruch 1, dadurch gekennzeichnet, daß jeder Verteilerzylinder (3) mit einem konzentrischen Verteilerkanal (35) versehen ist, dessen Enden (36) und (37) den Eingang und den Ausgang des Verteilers bilden, wobei die Verteilung radial mit Hilfe von auf verschiedenen Ebenen angeordneten Kanälen (38) erfolgt.

9. Multisynthetisierer für die Herstelung von Peptiden nach Anspruch 8, dadurch gekennzeichnet, daß die Verteilerzylinder (3) vorteilhaft acht an der Zahl sind, nämlich ein Zylinder zur Hauptverteilung der Lösungsmittel (3A), ein Zylinder zur Hauptverteilung des Stickstoffs (3B), ein Zylinder für die Verteilung des Niederdruck-Stickstoffs (3C) in Richtung Öffnung (16) der volumetrischen Dosierer (1), und für den Luft- und Stickstoffablaß aus der gleichen Öffnung (16), ein Zylinder für die Verteilung des Niederdruck-Stickstoffs (3D) in Richtung Öffnung (29) der chemischen Reaktoren (2) und für den Luft- und Stickstoffablaß aus der gleichen Öffnung (29), ein Zylinder zur Hauptverteilung für den Ablaß (3E) von Luft- und Stickstoffdampf in Richtung einer Abzughaube (44), ein Zylinder zur Hauptverteilung für den Ablaß der Abfallprodukte (3F) in Richtung Abfallverwertung (42), ein Zylinder zur Hauptverteilung (3G) für Stickstoff sehr geringen Drucks in Richtung der Öffnung (30) der chemischen Reaktoren (2) und ein Zylinder für die Verteilung von Stickstoff zur Entleerung des chemischen Reaktors (2).

10. Multisynthetisierer für die Herstellung von Peptiden nach Anspruch 1, dadurch gekennzeichnet, daß der Lösungsmittel-Selektor (4) von einem konzentrischen Verteilerkanal (39) durchquert wird, dessen eines Ende (40) durch Schläuche (10) und Verbindungsstücke (11, 11') mit einem der Verteilerzylinder (3) und dessen anderes Ende (41) über Schläuche (10) und Anschlußstücke (11, 11') mit einer Vorrichtung zur Abfallverwertung (42) verbunden ist, und daß die verschiedenen Lösungsmittel dem Verteilerkanal (39) über Kanäle (43) mit mittels Ventilen (12) gesteuerter Öffnung zugeführt werden und jeweils entsprechend der Steigung eines Schraubengangs auf verschiedenen Ebenen angeordnet sind.

11. Syntheseverfahren für die Herstellung von Peptiden unter Verwendung der Vorrichtung nach einem der Ansprüche 1 bis 10, dadurch gekennzeichnet, daß es darin besteht, gleichzeitig und unabhängig von einander die Synthese einer variablen Anzahl von Peptiden in verschiedenen Mengen, identischen oder verschiedenen Sequenzen, nach einer für jeden Zyklus gemeinsamen, reproduzierbaren Synthesetechnik herzustellen, entweder ausgehend von einer Kupplung Harz-Aminosäure, die man jedem chemischen Reaktor (2) zuführt, oder indem man eine solche Kupplung für jede Einheit volumetrischer Lösungsmitteldosierer (1)-chemischer Reaktor (2) herstellt, dann die Amino-Funktion N-α jeder Aminosäure entschützt, indem man die Schutzgruppe t-Butyloxycarbonyl durch Säurehydrolyse entfernt und die in Form von Salz gewonnene Amino-Gruppe neutralisiert, die zweite geschützte Aminosäure an das Harz kuppelt, deren Amino-Funktion N-α entschützt und diesen Zyklus für jede herzustellende Peptide, von identischer oder unterschiedlicher Sequenz, so oft wiederholt, wie die Zahl der in dem Harz einzufügenden Aminosäuren beträgt.

12. Verfahren für die Herstellung von Peptiden nach Anspruch 11, dadurch gekennzeichnet, daß zur Herstellung der Harz-Aminosäuren-Kupplung die folgenden Schritte für jede Einheit volumentrischer Lösungsmitteldosierer (1)-chemischer Reaktor (2) mit Hilfe der Steuerung des programmierbaren Automaten (7) oder des Mikrocomputers durchgeführt werden :

manuelle Zufuhr von Harz durch die Öffnung (28) ;

Einspritzen eines stickstoffgefüllten Lösungsmittels, das heißt von Dichloromethan, durch die Öffnung (26) ;

Zufuhr von unter sehr niedrigem Druck stehenden Stickstoff durch die Öffnung (30) zum Zweck des Bewegens mittels Durchperlen etwa eine Minute lang, um das Harz mittels Bewegen des Lösungsmittels und des Harzes zu waschen und aufgehen zu lassen ;

Zufurh von Stickstoff unter Druck durch die Öffnung (26), um das Lösungsmittel durch die Öffnung (30) zu abzulassen ;

Wiederholung dieser drei letzten Durchgänge bis zur Gewinnung von reinem Harz ;

manuelle Zufuhr der Schutzgruppe t-Butyloxycarbonyl-Aminosäure durch die Öffnung (28) ;

Einspritzen eines stickstoffgefüllten Lösungsmittels, das heißt von Dichloromethan, durch die Öffnung (26) ;

Zufuhr von unter sehr niedrigem Druck stehenden Stickstoff durch die Öffnung (30), etwa vier Stunden lang, für das Bewegen mittels Druchperlen unter wiederholter manueller Zugabe von Kuppelmedien und/oder Aktivierern durch die Öffnung (28) ;

Zufuhr von Stickstoff unter Druck durch die Öffnung (26) zum Ablassen des Lösungsmittels durch die Öffnung (30) ;

Einspritzen eines stickstoffgefüllten Lösungsmittels, das heißt von Dichloromethan, durch die Öffnung (26) ;

Zufuhr von unter sehr niedrigem Druck stehenden Stickstoff durch die Öffnung (30), etwa eine Minute lang, für das Bewegen mittels Durchperlen, um die Harz-Aminosäurekupplung zu waschen ;

Zufurh von Stickstoff unter Druck durch die Öffnung (26) zum Ablassen des Lösungsmittel durch die Öffnung (30) ;

Wiederholung dieser letzten drei Operationen bis zur Gewinnung einer sauberen Harz-Aminosäure-Kupplung.

13. Verfahren für die Herstellung von Peptiden nach einem der Ansprüche 11 und 12, dadurch gekennzeichnet, daß für das Entschützen der Amino-Funktion N-$\alpha$ der geschützten Aminosäure durch Entfernen der Schutzgruppe t-Butyloxycarbonyl durch Säurehydrolyse, die folgenden Schritte für jede Einheit volumetrischer Lösungsmitteldosierer (1)-chemischer Reaktor (2) mit Hilfe der Steuerung des programmierbaren Automaten (7) oder des Mikrocomputers durchgeführt werden :

Einspritzen unter Stickstoffdruck durch die Öffnung (26) einer Mischung aus 35 % Dichloromethan und 65 % Trifluoroessigsäure ;

Zufuhr von unter sehr niedrigem Druck stehenden Stickstoff durch die Öffnung (30), etwa eine Minute lang, für das Bewegen mittels Durchperlen ;

Zufuhr von Stickstoff unter Druck durch die Öffnung (26) zum Ablassen der Dichloromethan-Trifluoroessigsäure durch die Öffnung (30) ;

Einspritzen der Mischung aus stickstoffgefülltem Dichloromethan- und Trifluoroessigsäure zu gleichen Teilen durch die Öffnung (26) ;

Zufuhr, etwa 13 Minuten lang, von unter sehr niedrigem Druck stehenden Stickstoff durch die Öffnung (30) für das Bewegen mittels Durchperlen ;

Zufuhr von Stickstoff unter Druck durch die Öffnung (26) zum Ablassen der Mischung Dichloromethan-Trifluoroessigsäure durch die Öffnung (30) ;

Einspritzen eines stickstoffgefüllten Lösungsmittels, das heißt von Dichloromethan, durch die Öffnung (26) ;

Zufuhr von unter sehr niedrigem Druck stehenden Stickstoff durch die Öffnung (30), etwa eine Minute lang, für das Bewegen mittels Durchperlen, um die entschützte Harz-Aminosäure-Kupplung zu waschen ;

Zufuhr von Stickstoff unter Druck durch die Öffnung (26) zum Ablassen des Lösungsmittels durch die Öffnung (30) ;

Wiederholung dieser drei letzten Operationen ;

Einspritzen eines stickstoffgefüllten Lösungsmittels, das heißt von Dimethylformamid durch die Öffnung (26) um die Reste der Trifluoroessigsäure zu entfernen ;

Zufuhr von unter sehr geringem Druck stehenden Stickstoff durch die Öffnung (30), einige Sekunden lang, für das Bewegen mittels Durchperlen ;

Zufuhr von Stickstoff unter Druck durch die Öffnung (26) zum Ablassen des Lösungsmittels durch die Öffnung (30) ;

Einspritzen eines Lösungsmittels, das heißt von Dichloromethan, durch die Öffnung (26) ;

Zufuhr von unter sehr geringem Druck stehenden Stickstoff durch die Öffnung (30), etwa eine Minute lang, für das Bewegen mittels Durchperlen ;

Zufuhr von Stickstoff unter Druck durch die Öffnung (26) zum Ablassen des Lösungsmittels durch die Öffnung (30) ;

Einspritzen eines Lösungsmittels, d. h. von Dimethylformamid, durch die Öffnung (26) ;

Zufuhr von unter sehr niedrigem Druck stehenden Stickstoff, durch die Öffnung (30) etwa eine Minute lang für Bewegung mittels Durchperlen ;

Zufuhr von Stickstoff unter Druck durch die Öffnung (26) zum Ablassern des Lösungsmittels durch die Öffnung (30) ;

Wiederholung dieser letzten Operation.

14. Verfahren für die Herstellung von Peptiden nach Anspruch 11, dadurch gekennzeichnet, daß zur Neutralisierung der in Form von Salz gewonnenen Amino-Gruppe, folgende Schritte für jede Einheit volumetrische Lösungsmittel (1)-chemischer Reaktor (2) mit Hilfe der Steuerung des progammierbaren Automaten (7) oder des Mikrocomputers durchgeführt werden :

Einspritzen einer stickstoffgefüllten Mischung aus 10 % Diisopropyläthylamin und 90 % Dichloromethan oder Dimethylformamid durch die Öffnung (26) ;

Zufuhr von Stickstoff unter sehr geringem Druck durch die Öffnung (30), etwa eine Minute lang, für das Bewegen mittels Durchperlen ;

Zufuhr von Stickstoff unter Druck durch die Öffnung (26) zum Ablassen der Diisopropyläthylamin-Dichloromethan-Mischung oder der Diisopropyläthylamin-Dimethylformamid-Mischung durch die Öffnung (30) ;

Wiederholung dieser letzten Operation ;

Einspritzen einer stickstoffgefüllten Lösung, das heißt von Dichloromethan oder Dimethylformamid durch die Öffnung (26) ;

Zufuhr von Stickstoff unter sehr geringem Druck durch die Öffnung (30), etwa eine Minute lang, für das Bewegen mittels Durchperlen ;

Zufuhr von Stickstoff unter Druck durch die Öffnung (26) zum Ablassen des Lösungsmittels durch die Öffnung (30) ;

dreifache Wiederholung dieser letzten Operation für das Waschen der Harz-Aminosäure-Kupplung ;

manuelle Entnahme, durch die Öffnung (28), eine Probe der Kupplung Harz-Aminosäure zur Überprüfung der Reaktion der Entschützung mit Hilfe eines Tests auf Ninhydrin.

15. Verfahren für die Herstellung von Peptiden nach Anspruch 11, dadurch gekennzeichnet, daß, um die zweite geschützte Aminosäure an die gewonnene Kupplung Harz-Aminosäure zu kuppeln, die folgenden Schritte für jede Einheit volumetrischer Lösungsmitteldosierer (1)-chemischer Reaktor (2) mit Hilfe der Steuerung des programmierbaren Automaten (7) oder des Mikrocomputer durchgeführt werden :

manuelle Zufuhr der aktivierten oder nicht aktivierten geschützten Aminosäure durch die Öffnung (28) ;

Einspritzen eines stickstoffgefüllten Lösungsmittels, das heißt von Dichloromethan oder Dimehtylformamid durch die Öffnung (26) ;

Zufuhr von Stickstoff unter sehr geringem Druck durch die Öffnung (30), zwischen 10 und 60 Minuten lang, für das Bewegen mittels Durchperlen mit manueller Zufuhr von Kuppelmitteln und/oder Aktivieren durch die Öffnung (28) ;

manuelle Entnahme durch die Öffnung (28) einer Probe der gewonnenen Harz-Aminosäure-Kupplung und Test auf Ninhydrin ;

entweder Zufuhr von Stickstoff unter Druck durch die Öffnung (26) zum Ablassen des Lösungsmittels durch die Öffnung (30), wenn der während der vorheringen Operation ausgeführte Test negativ ist, oder Wiederaufnahme der Operationen zu Beginn der Neutralisierung der in Form von Salz gewonnenen Amino-Gruppe, wenn der während der vorigen Operation ausgeführte Test positif ist ;

Einspritzen eines Lösungsmittels unter Druck, das heißt von Dichlormethan, durch die Öffnung (26) ;

Zufuhr von Stickstoff unter sehr geringem Druck durch die Öffnung (30), etwa eine Minute lang, für das Bewegen mittels Durchperlen, um die gewonnene Kupplung Harz-Aminosäure zu waschen ;

Zufuhr von Stickstoff unter Druck durch die Öffnung (26) zum Ablassen des Lösungsmittels durch die Öffnung (30) ;

dreifache Wiederholung dieser letzten Operation bis zur Erzielung einer sauberen Verbindung.

16. Verfahren für die Herstellung von Peptiden nach einem der Ansprüche 11 bis 15, dadurch gekennzeichnet, daß die Volummenmessung des in jedem chemischen Reaktor (2) durch die Öffnung (26) einzuspritzenen Lösungsmittels mittels der Steuerung des programmierbaren Automaten (7) oder des Mikrocomputers folgendermaßen durchgeführt wird ;

Öffnung der Zulaufventile (120) für das selektierte Lösungsmittel und (120') für den Zulauf von selektiertem Stickstoff ;

Öffnung des Zulaufventils (121) der Öffnung (16) des volumetrischen Lösungsmitteldosierers (1) ;

Öffnung des Entlüftungsventils (122) des Verteilerzylinders (3C) ;

Einspritzen einer stickstoffgefüllten Lösung durch die Öffnung (16) des volumetrischen Dosierers (1) bis zum Auffinden des Niveaus durch den Stab der optoelektronischen Detektoren (25) ;

Schließen des Zulaufventils (121) der Öffnung (16) des volumetrischen Lösungsmitteldosierers (1) ;

Abgleichung des vorherbestimmten Volumens für jede Einheit volumetrischer Lösungsmitteldosierer (1) — chemischer Reaktor (2) ;

Schließen der Zulaufventile (120) und (120') der Kupplung Lösungsmittel-selektierter Stickstoff.

17. Verfahren für die Herstellung von Peptiden nach einem der Ansprüche 11 bis 15, dadurch gekennzeichnet, daß das Einspritzen des Lösungsmittels in jeden chemischen Reaktor (2) mittels der Steuerung des programmierbaren Automaten (7) oder des Mikrocomputers folgendermaßen durchgeführt wird :

Öffnen des Zulaufventils (123) für Stickstoff des Verteilerzylinders (3C) in der Weise, das durch die Öffnung (16) des volumetrischen Dosierers (1) die in dem Hauptzylinder (13) und in der Meßsäule (14) des volumetrischen Lösungsmitteldosierers (1) enthaltene Lösung unter Druck gesetzt wird ;

unter Unterdruck setzen des chemischen Reaktors (2) durch Öffnen des Druckminderventils (125) der Öffnung (29) über den Verteilerzylinder (3D) ;

Öffnen des Zulaufventils (126) der Öffnung (26) ;

unter Unterdruck setzen, etwa 5 Sekunden lang, des volumetrischen Dosierers (1) durch Öffnung des Entlüftungsventils (122) des Verteilerzylinders (3C).

18. Verfahren für die Herstellung von Peptiden nach einem der Ansprüche 11 bis 15, dadurch gekennzeichnet, daß das Bewegen mittels Durchperlen in jedem chemischen Reaktor (2) mittels der Steuerung des programmierbaren Automaten (7) oder des Mikrocomputers folgendermaßen durchgeführt wird :

unter Unterdruck setzen des Reaktors durch Öffnung des Druckminderventils (125) der Öffnung (29) über den Verteilerzylinder (3D) ;

Verweilzeit von 5 Sekunden zur Druckminderung des chemischen Reaktors (2) ;

Öffnen der Hauptventile (127, 127') zur Verteilung von Stickstoff unter sehr geringem Druck oberhalb des Verteilerzylinders (3G) ;

Öffnen des Zulaufventils für Stickstoff (130) von der Öffnung (30) ;
Öffnen des Ventils (128) das den Überdruckimpuls eine halbe Sekunde lang überprüft ;
Schließen des Ventils (128) ;
gesteuerte Verteilung von Stickstoff unter sehr geringem Druck für ein Bewegen mittels Durchperlen durch die Öffnung (30) ;
Schließen des Ventils (130) für die Zufuhr von Stickstoff von der Öffnung (30) ;
Verweilzeit von 200 ms von Beginn der Entleerung.

19. Verfahren für die Herstellung von Peptiden nach einem der Ansprüche 11 bis 15, dadurch gekennzeichnet, daß die Entleerung jedes chemischen Reaktors (2) mittels Steuerung des programmierbaren Automaten (7) oder des Mikrocomputers folgendermaßen durchgeführt wird :
Öffnen des Ventils (126') für die Zufuhr von Stickstoff von der Öffnung (26) des Verteilerzylinders (3H) ;
Öffnen des Ventils für Abfallprodukte (124) des Verteilerzylinders (3F) ;
Öffnen des Ventils zum Ablaß der Lösungsmittel (131) der Öffnung (30) ;
unter Unterdruck setzen des Reaktors durch Öffnen des Druckminderventils (125) der Öffnung (29) über den Verteilerzylinder (3D) ;
Verweilzeit von 5 Sekunden zur Druckminderung des chemischen Reaktors (2) ;

20. Verfahren für die Herstellung von Peptiden nach einem der Ansprüche 11 bis 15, dadurch gekennzeichnet, daß das Ausspülen des Lösungsmittelselektors (4) von den Restsegmenten des vorherigen Lösungsmittels über die Steuerung des programmierbaren Automaten (7) oder des Mikrocomputers folgendermaßen durchgeführt wird :
Öffnen des Ventils für den Zulauf (123) von Stickstoff des Verteilerzylinders (3C) ;
Öffnen des Abfall-Ventils (129) des Lösungsmittel-Selektors (4) ;
Öffnen des Zulaufventils (121) der Öffnung (16).

21. Verfahren für die Herstellung von Peptiden nach einem der Ansprüche 11 bis 15, dadurch gekennzeichnet, daß das Füllen der Lösungsmittelflaschen (6) mittels der Steuerung des programmierbaren Automaten (7) oder des Mikrocomputers folgendermaßen durchgeführt wird :
Schließen des Niederdruckventils (132) des Verteilerzylinders (3B) ;
Öffnen des Entlüftungsventils (133) des Verteilerzylinders (3B) ;
Öffnen des Ventils (120') der in Betracht kommenden Lösungsmittelflasche ;
manuelles Füllen der Flasche (6) ;
Schließen des Ventils (120') der entsprechenden Lösungsmittelflasche ;
Schließen des Entlüftungsventils (133) des Verteilerzylinders (3B) ;
Öffnen des Niederdruckventils (132) des Verteilerzylinders (3B).

Fig-1

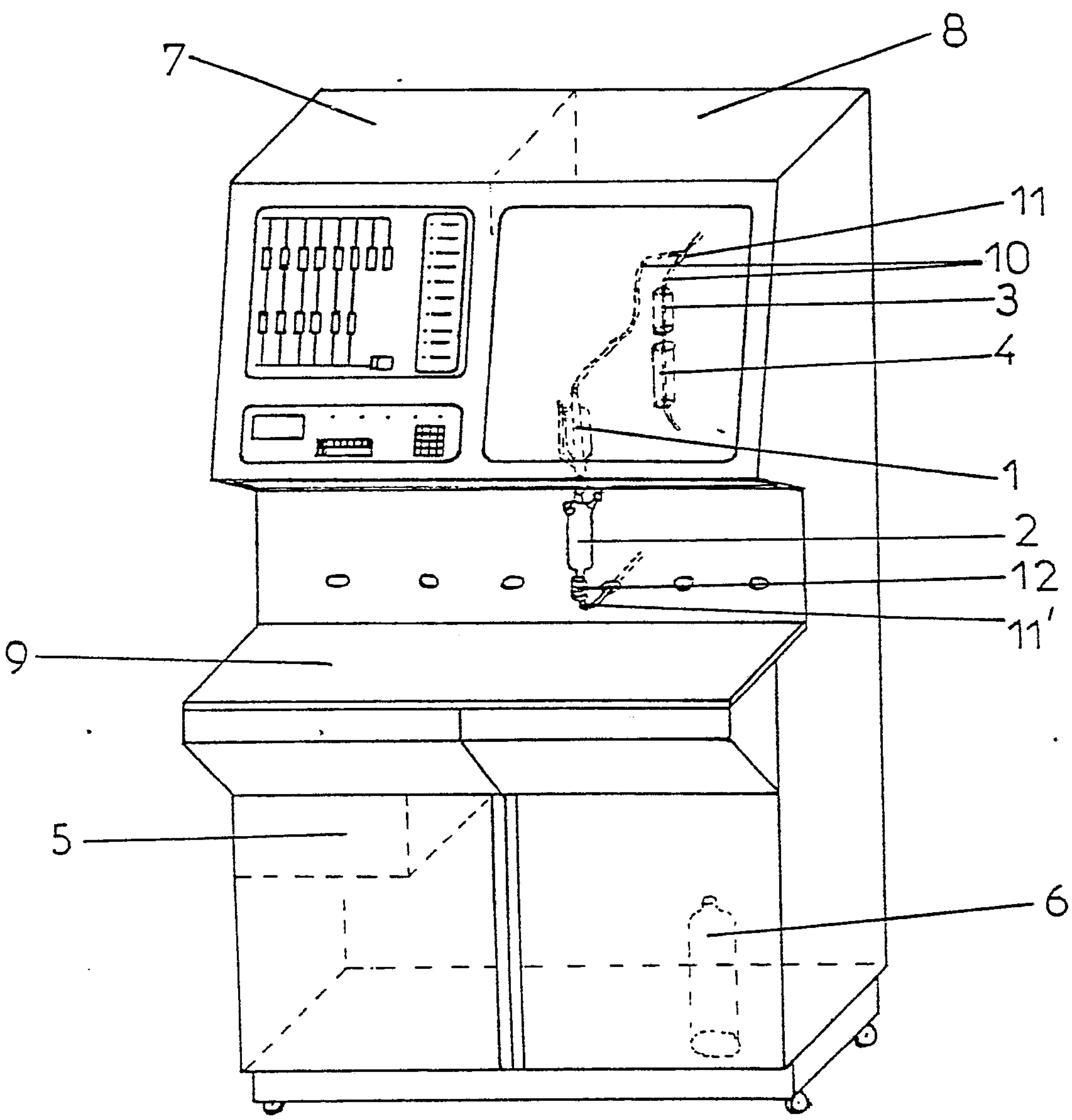

Fig. 2

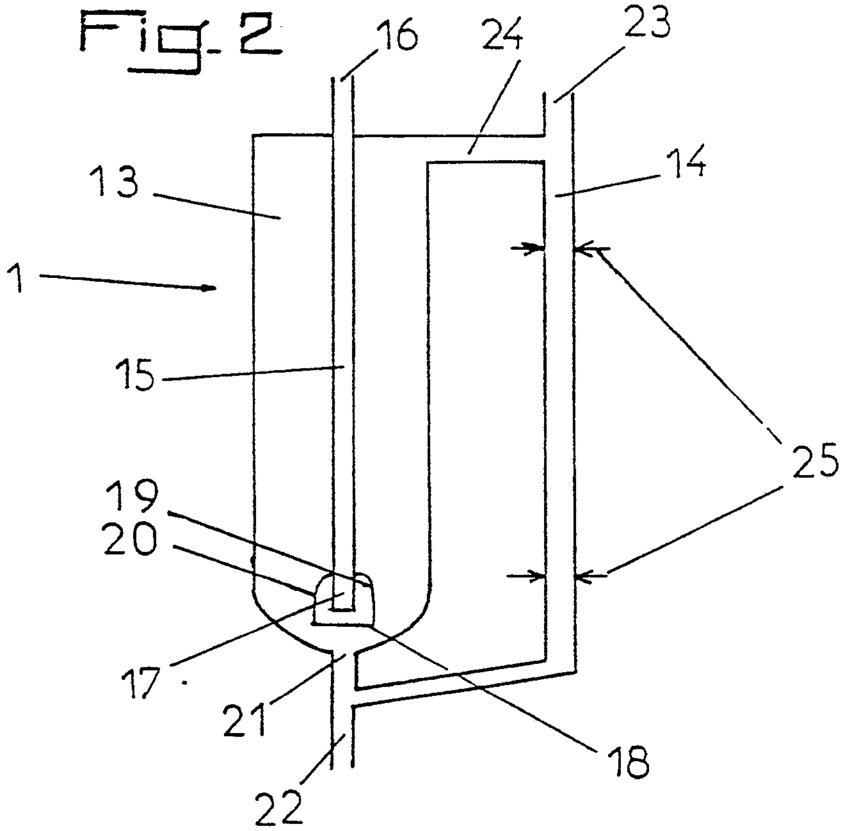


Fig. 5

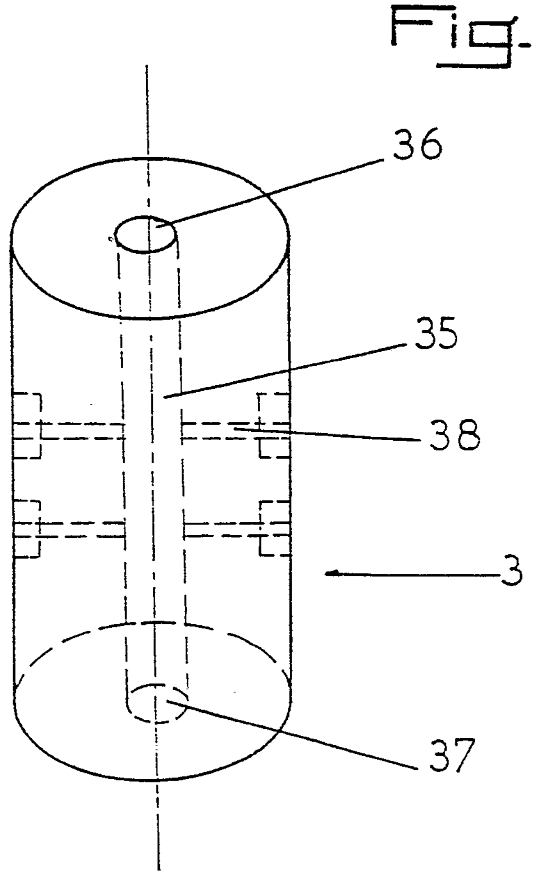

Fig-3

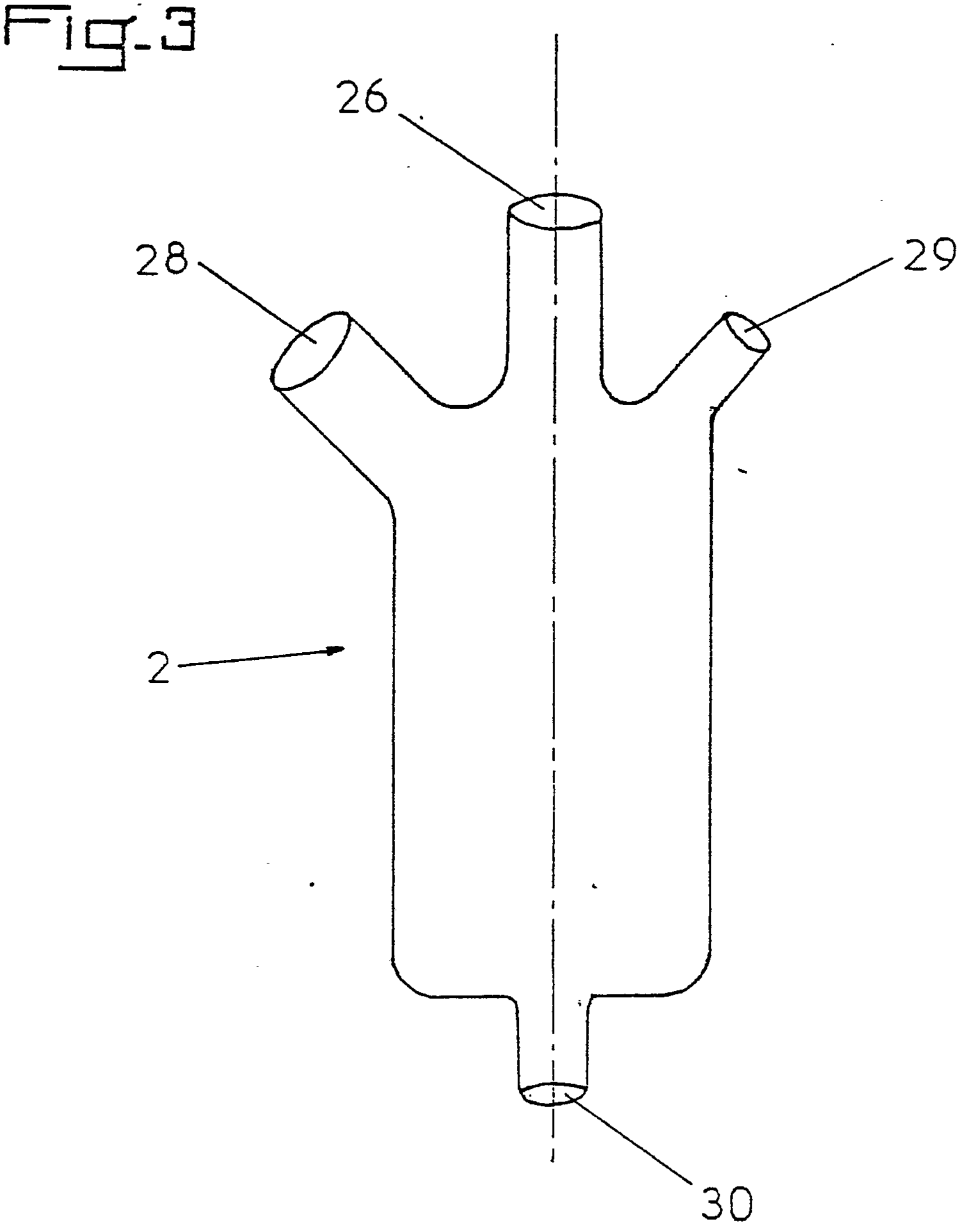

Fig.4

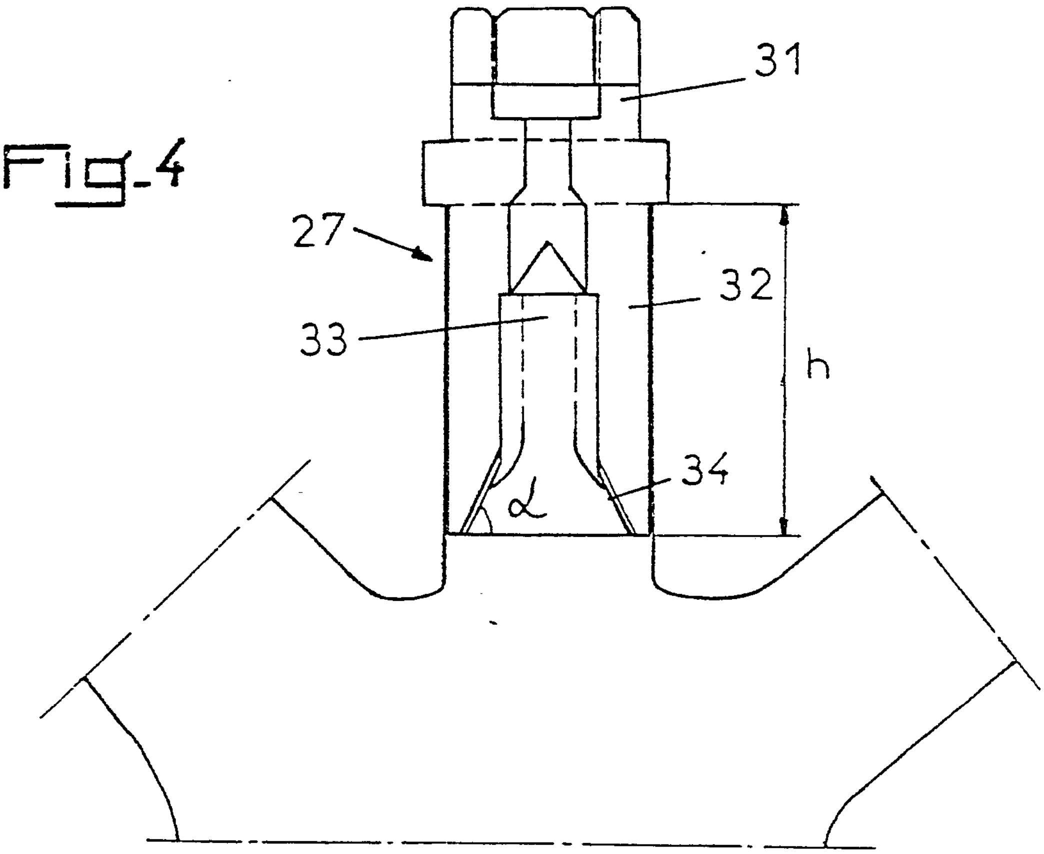


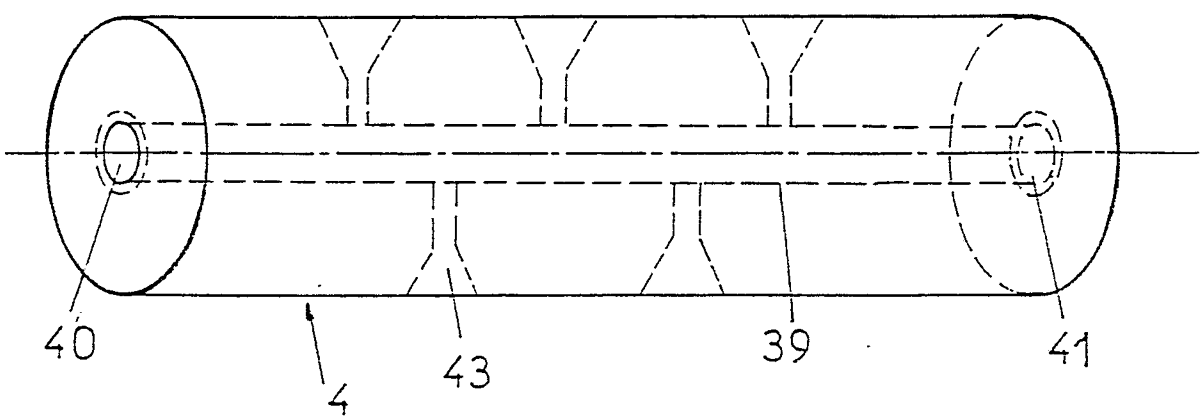


Fig.6

Fig.7

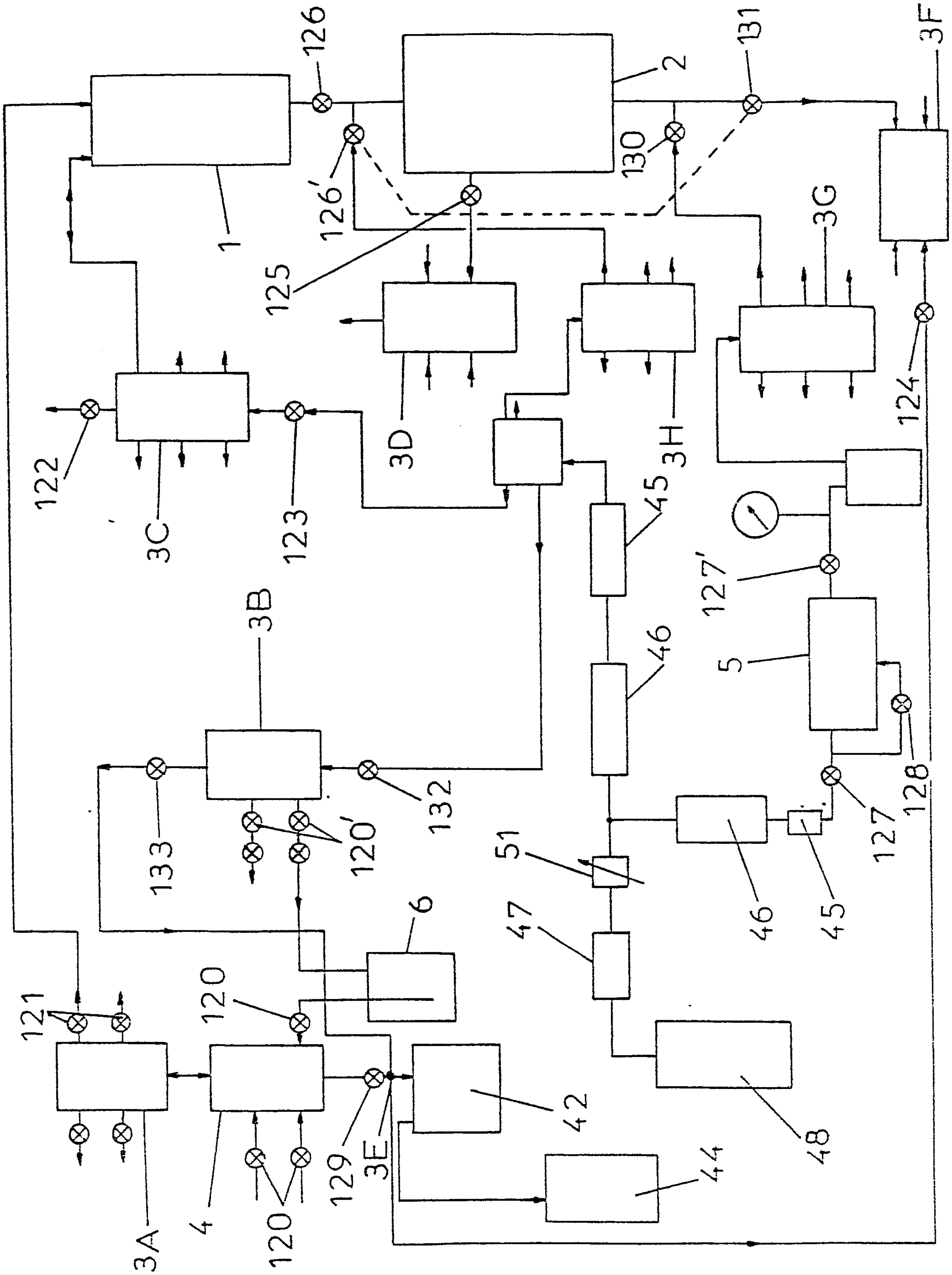
1
2
3A
3B
3C
3D
3E
3F
3G
3H
4
5
6
42
44
45
46
47
48
51
120
120'
121
122
123
124
125
126
126'
127
127'
128
129
130
131
132
133

Fig. 8

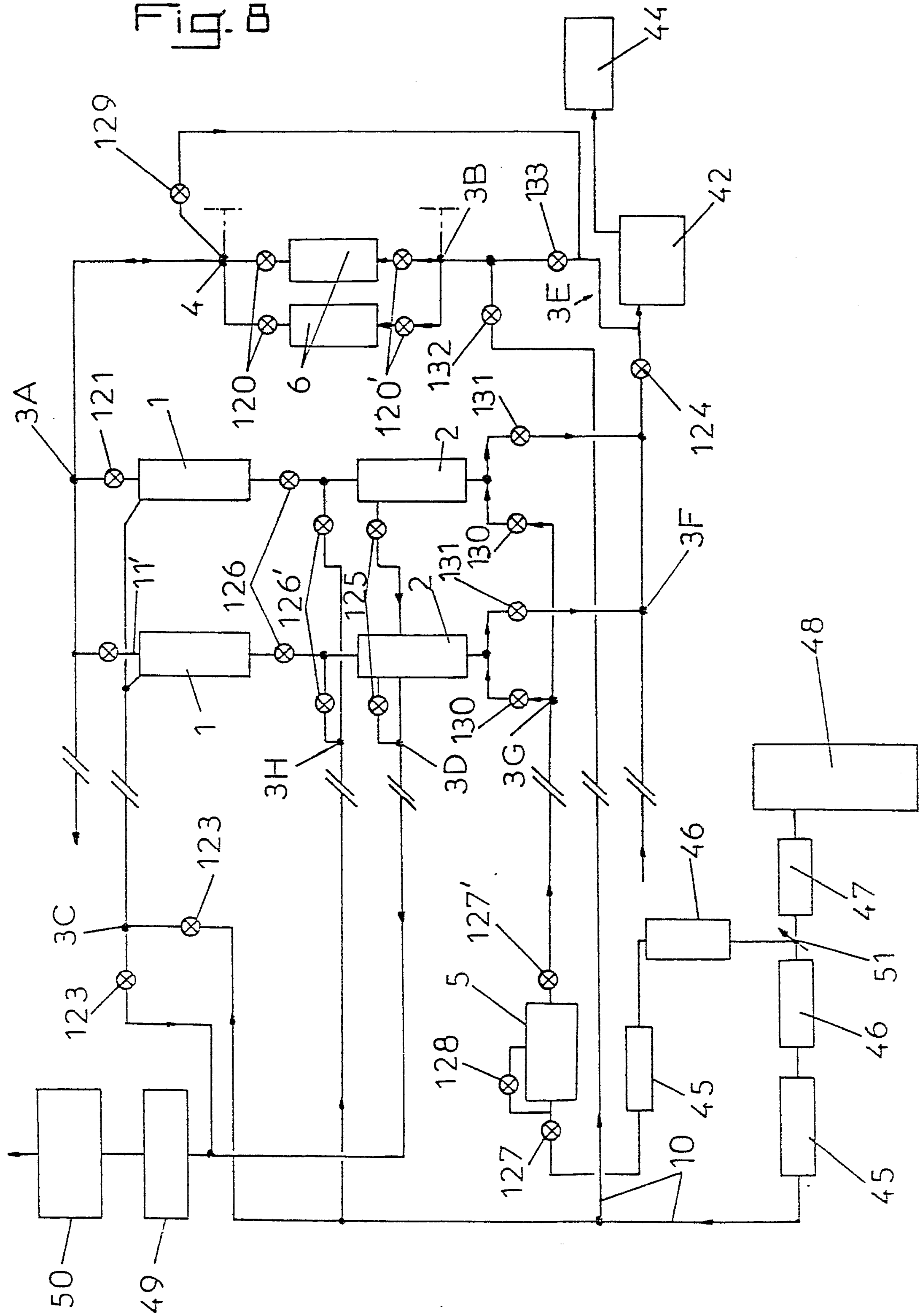
44
129
3B
133
42
4
3E
120
6
120'
132
124
3A
121
1
2
131
11'
126
126'
125
130
3F
2
131
130
1
3H
3D
3G
48
123
46
3C
127'
47
51
123
5
46
128
45
10
127
45
50
49